# EUROPEAN PATENT SPECIFICATION

(11) **EP 2 491 135 B1**
(45) Date of publication and mention of the grant of the patent: **30.11.2016**
(21) Application number: 10774167.0
(22) Date of filing: 19.10.2010
(51) Int. Cl.: C12Q 1/68

(54) **Method for streptococcus pneumoniae diagnosis and serotyping**
Verfahren zur Diagnose und Serotypisierung von Streptokokkenpneumonie
Procédé pour le diagnostic et le sérotypage de Streptococcus pneumoniae

(30) Priority: 19.10.2009 EP 09173437
(43) Date of publication of application: 29.08.2012
(73) Proprietor: Azienda Ospedaliero-Universitaria Meyer, 50139 Firenze (IT)
(72) Inventor: AZZARI, Chiara, I-50139 Firenze (IT); MORIONDO, Maria, I-50139 Firenze (IT); RESTI, Massimo, I-50139 Firenze (IT)
(74) Representative: Capasso, Olga
(86) International application number: PCT/EP2010/065733
(87) International publication number: WO 2011/048104

(56) References cited:
- WO-A2-2007/106407
- TARRAGÓ D ET AL: "Identification of pneumococcal serotypes from culture-negative clinical specimens by novel real-time PCR." CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES SEP 2008, vol. 14, no. 9, September 2008 (2008-09), pages 828-834, XP002560716 ISSN: 1469-0691
- SAHA SAMIR K ET AL: "Identification of serotype in culture negative pneumococcal meningitis using sequential multiplex PCR: implication for surveillance and vaccine design." PLOS ONE 2008, vol. 3, no. 10, 2008, page E3576, XP002560717 ISSN: 1932-6203
- WANG QUAN ET AL: "Development of a DNA microarray to identify the Streptococcus pneumoniae serotypes contained in the 23-valent pneumococcal polysaccharide vaccine and closely related serotypes" JOURNAL OF MICROBIOLOGICAL METHODS, vol. 68, no. 1, January 2007 (2007-01), pages 128-136, XP22731910 ISSN: 0167-7012
- PAI REKHA ET AL: "Sequential multiplex PCR approach for determining capsular serotypes of Streptococcus pneumoniae isolates." JOURNAL OF CLINICAL MICROBIOLOGY JAN 2006, vol. 44, no. 1, January 2006 (2006-01), pages 124-131, XP002560719 ISSN: 0095-1137
- LAWRENCE ELLIOT R ET AL: "Evaluation of semiautomated multiplex PCR assay for determination of Streptococcus pneumoniae serotypes and serogroups." JOURNAL OF CLINICAL MICROBIOLOGY FEB 2003, vol. 41, no. 2, February 2003 (2003-02), pages 601-607, XP002560720 ISSN: 0095-1137
- BRITO D A ET AL: "Serotyping Streptococcus pneumoniae by multiplex PCR." JOURNAL OF CLINICAL MICROBIOLOGY JUN 2003, vol. 41, no. 6, June 2003 (2003-06), pages 2378-2384, XP002560721 ISSN: 0095-1137
- DATABASE EMBL [Online] 30 June 2005 (2005-06-30), "Streptococcus pneumoniae strain 519/43 (serotype 1)." XP002560722 retrieved from EBI accession no. EMBL:CR931632 Database accession no. CR931632 -& BENTLEY STEPHEN D ET AL: "Genetic analysis of the capsular biosynthetic locus from all 90 pneumococcal serotypes." PLOS GENETICS MAR 2006, vol. 2, no. 3, March 2006 (2006-03), page E31, XP002560723 ISSN: 1553-7404
- WITTWER C T ET AL: "Real-time multiplex PCR assays" 1 December 2001 (2001-12-01), METHODS : A COMPANION TO METHODS IN ENZYMOLOGY, ACADEMIC PRESS INC., NEW YORK, NY, US, PAGE(S) 430 - 442 , XP002265718 ISSN: 1046-2023 the whole document
- CHIARA AZZARI ET AL: 'Realtime PCR Is More Sensitive than Multiplex PCR for Diagnosis and Serotyping in Children with Culture Negative Pneumococcal Invasive Disease' PLOS ONE vol. 5, no. 2, E9282, 19 February 2010, pages 1 - 7, XP055191385 DOI: 10.1371/journal.pone.0009282
- CHIARA AZZARI ET AL: 'Potential serotype coverage of three pneumococcal conjugate vaccines against invasive pneumococcal infection in Italian children' VACCINE vol. 30, no. 16, 01 March 2012, pages 2701 - 2705, XP055191384 DOI: 10.1016/j.vaccine.2011.12.008 ISSN: 0264-410X

## Description

### FIELD OF THE INVENTION

The present invention relates to a method for the typing of multiple Pneumococcal serotypes directly on clinical samples. The method is based on realtime PCR amplification of specific genomic regions by using identified combinations of primer/probe sets.

### BACKGROUND ART

At present, what is known about pneumococcal serotype distribution in invasive infections or carriage, is based mostly on cultural isolate typing [24].

Pneumococcal serogroup and serotype identification is currently performed by using large panels of expensive antisera by capsular swelling reaction [9,10,13]. However, cross-reactions between serotypes can occur and some strains are difficult to serotype or remain non-serotypable [11-14]. At present, capsule thickness is a serious problem in serologic serotyping. Antisera cannot react correctly with capsular structures of certain pneumococcal serotypes such as serotype 3. This problem can be overcome using the method of the present invention since DNA extraction and amplification is not disturbed by capsule thickness. Serotyping of isolates by molecular methods such as multiplex sequential PCR (MS-PCR) has been previously performed [2] and high sensitivity and excellent concordance with standard cultural methods has been demonstrated [2].

However, it is well known that cultures may give false-negative results because of small sample volume, previous antibiotic therapy, or unsatisfactory conditions of transport and storage which can impair the viability of pathogens. In all the cases where culture growth is not obtained, serotyping cannot be performed neither with Quellung reaction nor with molecular methods.

The authors of the present invention previously demonstrated that MS-PCR can be used directly on clinical samples for *Streptococcus Pneumoniae* serotyping, thus giving results where cultural methods fail [3]. The method disclosed in the application WO 2009/024844 used multiplex sequential PCR directly on clinical sample for the diagnosis and serotyping of Streptococcus pneumoniae. The method has a sensitivity and specificity significantly higher than standard cultural methods known, at that time, as the gold standard for microbiological diagnosis. The present invention aims at further improving the diagnosis and serotyping of Streptococcus pneumoniae infections.

MS-PCR techniques can be further improved using realtime PCR. At present, among molecular methods, realtime PCR has probably the best performance in terms of sensitivity, specificity and rapidity. The use of specific procedures and primer/probes sets makes the test extremely specific and sensitive. Moreover, detection of amplification products during the amplification step itself makes the test the most rapid in molecular biology. As for sensitivity, it has been previously demonstrated in different research fields that realtime PCR sensitivity is higher than that of standard PCR and evaluation on agarose gel [19]. In fact, realtime PCR has been used over a large spectrum of fields in biological research [25-27]. In recent years, molecular techniques have been applied successfully to the identification of infectious agents. The knowledge of DNA/RNA sequences of most bacteria allow to obtain an etiologic diagnosis and bacterial serotyping [1,2] even where standard culture methods fail [3, 4]. The need for viable bacteria as well as technical expertise requirements are serious drawbacks of the culture detection-system. In addiction, autolysis in culture media and antibiotic therapy often performed on an outpatient basis at the beginning of febrile illnesses, make diagnosis and serotyping with culture methods more difficult [4, 5].

Molecular methods, based on DNA detection, do not require viable bacteria so that bacterial detection can be obtained even after starting antimicrobic treatment [4]. For this reason molecular methods have higher sensitivity in reaching an etiologic diagnosis, greatly reduce the proportion of false-negative results and can be used as a better tools to obtain an epidemiologic picture in different areas in the world.

Among the most common bacterial pathogens, *Streptococcus Pneumoniae* is considered the leading cause of invasive bacterial infections both in children and in elderly people [6]. The organism causes at least 1.6 million deaths each year and is responsible for several invasive infections [7].

Capsular polysaccharide helps to differentiate 91 distinct serotypes of *Streptococcus Pneumoniae* [8]. Pneumococcal serotyping is currently performed by using capsular swelling (Quellung) reaction, considered the traditional 'gold standard', [9-10]. However, cross-reactions between serotypes can occur and some strains remain non-serotypable [11-14]. At present, strains with a thick capsule such as serotype 3 cannot be easily typed because specific antisera are prevented from reaching specific epitopes on the capsule itself. This problem can be overcome using molecular methods since DNA extraction and amplification is not disturbed by capsule thickness.

Effective vaccines induce specific anti-capsular antibodies [15]. The available vaccine used in pediatric age induces protection against 7 major serotypes and new vaccines against 10 or 13 serotypes will be available in the next future. Individuating serotypes causing invasive infections is extremely important in order to understand pneumococcal epidemiology, plan correct programs of vaccination and evaluate the impact of the existing program. In particular, the correct analysis of serotype distribution is extremely important for decision makers in public health services; in fact it allows conscious decision on vaccine choice for the population in all countries of the world.

On the other hand, molecular typing is an important tool also in studies on pneumococcal carriage. At present, individuation and typing of pneumococci in nasopharyngeal swabs is obtained by a single colony method [16-17]. Presently, using this method only a single serotype per patient can be found and multiple colonization, which is a very frequent condition, cannot be detected [18]. Therefore, there is the need for a method of detection of S. pneumoniae serogroup that has improved sensitivity in respect to current methods and that is cost effective.

The authors of the present invention have previously described that multiplex sequential PCR (MS-PCR) performed directly on clinical samples for pneumococcal serotyping has a sensitivity significantly higher than that of culture [3]. Even though the possibility of pneumococcal serotyping has been greatly improved by MS-PCR methods, serotyping could be further optimized by the use of Realtime-PCR known, at present, as one of the most specific and sensitive molecular method [19-20]. For this reason, the use and the combination of primers/probes obtained from specific genomic regions of Streptococcus pneumoniae, the set up of precise procedures for amplification with RT-PCR and the direct use of clinical samples has led to the identification of an innovative method compared to prior art.

### DESCRIPTION OF THE INVENTION

The present invention relates to a method in which Realtime PCR is used, directly on clinical samples, for typing multiple Pneumococcal serotypes, known to be the most frequent ones both in children and adults worldwide. The present invention has an improved sensitivity compared to prior art methods for identifying and serotyping *Streptococcus Pneumoniae* both in blood or otherwise body fluids and in nasopharyngeal swabs, as shown by examples described in the following sections.

The present invention has several advantages: 1) it allows the serotyping of non-viable bacteria, therefore samples which do not yield cultural growth can be serotyped; 2) it is more sensitive than multiplex sequential PCR because it uses realtime PCR amplification instead of endpoint amplification and identification on agarose gels.

Moreover, the technique described in the present invention has also the highest specificity when compared to methods already described in the state of the art:
1) when compared to multiplex sequential PCR, the use of specific and unique primers and probe sets allow the unique identification of a single serotype.
2) Standard PCR techniques are lengthy including the time for completion of the amplification steps, and the time for agarose gel electrophoresis; therefore the individuation of serotype is not possible in less than 4 hour. Moreover, since the MS-PCR techniques include multiple steps, if the serotype is not individuated in the first step, additional amplification/electrophoresis steps (3-4hours/each) may be needed rendering the whole method even more time expensive.
3) Cultural detection of bacterial strains can be sometimes difficult. By contrast, the present method allows the identification of a unique strain in DNA of isolates or bacterial DNA of clinical samples with no error.
3) the present method displays improved sensitivity in respect of molecular methods using RT-PCR based on degenerate primers which do not show good sensitivity and may lead to ambiguous results, as demonstrated by examples described in the following sections (28).

The present invention allows the use of clinical samples directly in Realtime PCR for *Streptococcus Pneumoniae* serotyping. The sample taken from the human body may be any type of human biological tissue. Among these, blood, pleuric liquid and cefalorachidian liquor (CSF) are preferred. In the cases wherein an organ pathology (meningitis, pneumonia with pleuritis) is present, it is preferred that a sample be taken from the seat of infection (CSF or pleuric liquid). When these are not available and for the unlocalised forms (sepsis, bacteriaemia), whole blood is preferred, because the standard regulations for the sampling thereof and the displacement/freezing of the same are well known and practiced in the art, with equipment also known to the skilled in the art.

A further advantage of the method according to the present invention is that a sample of a biological tissue may be used, even if it has been sampled far away or however within a time interval greater than 1 day, while the maximum time for using the samples by culture is within one hour. Samples taken even 8 days earlier are still usable in the method according to the invention. The sample taken may optionally be frozen and defrosted at the time of analysis. Thus, with a method of freezing and defrosting of the sample, the presence and/or the serogroup and/or the serotype of a pathogen may be detected more than 8 days later and for an indefinite time. The only requirement of the method according to the invention is that the nucleic acid of the prokaryote remain intact and extractable and have not hydrolised or decomposed during the wait between the sampling and the beginning of the method.

The biological sample may be taken either in a test tube or absorbed as a spot on blotting paper, the blotting paper sheets being similar to those normally used for the Guthri test at birth, for the screening of hypothyroidism and phenylketonuria. An advantage of this method is that blood may be sampled also from a fingertip by capillary prick. Thus, the sampling may be done also at the patient's place of residence or in the doctor's office, since no sampling of venous blood is required. The sheet used for the spot (which is sterile prior to use), once used, must immediately be put away in a plastic sachet in order to avoid contaminations by environment germs and subsequently sent to the laboratory carrying out the test. In the contest of the method of the present invention, it is preferred to extract and use only DNA because RNA is labile and not preservable for a long time. Any method for extracting DNA from a sample of biological tissue known in the art may be used for the method of the invention. It is preferable to use a group of reagents or kits for extracting DNA comprising K protease, such as e.g. the QIAmp DNA mini kit, by Qiagen, Hilden, Germany. The use of K protease has shown, in comparative studies, a greater capability of retrieving bacterial DNA and said capability results in a greater sensitivity of the test.

The molecular method subject of the present invention can easily be used on stored biological samples, with no limitation of time. Therefore the method is suitable both for prospective and retrospective analysis allowing to give a complete epidemiological picture of invasive pneumococcal disease.

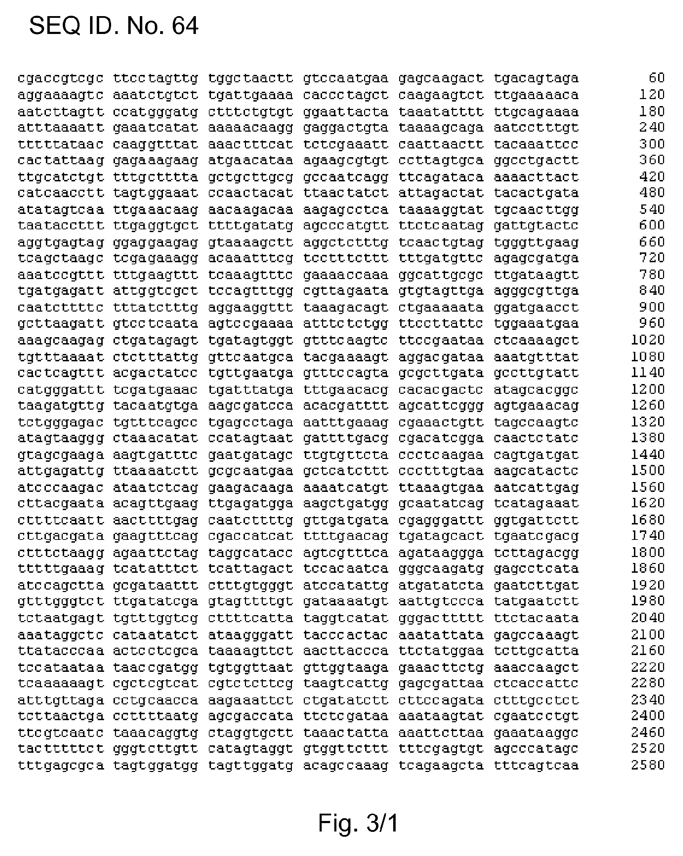

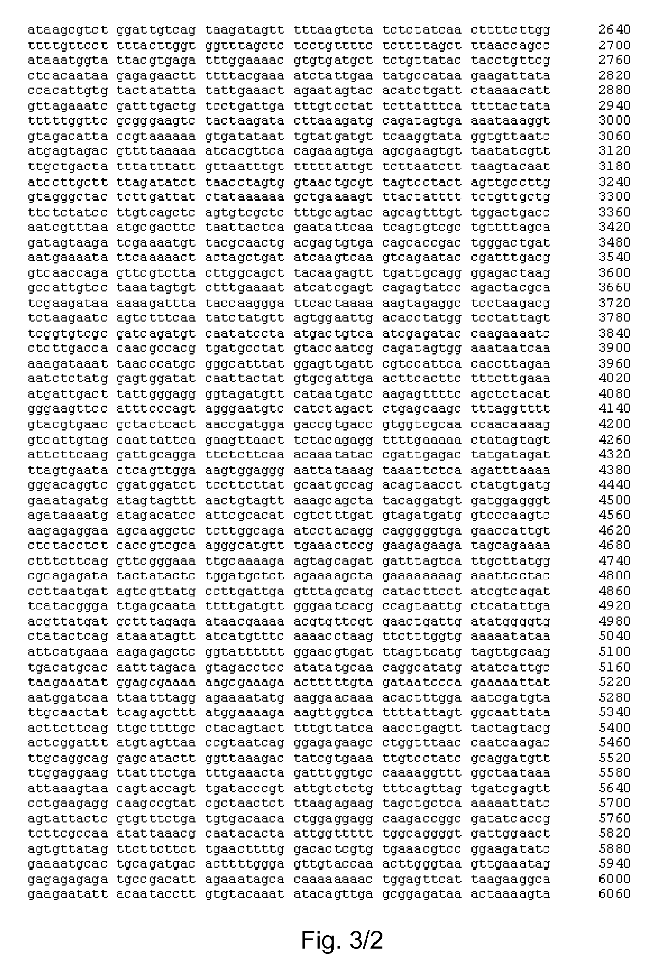

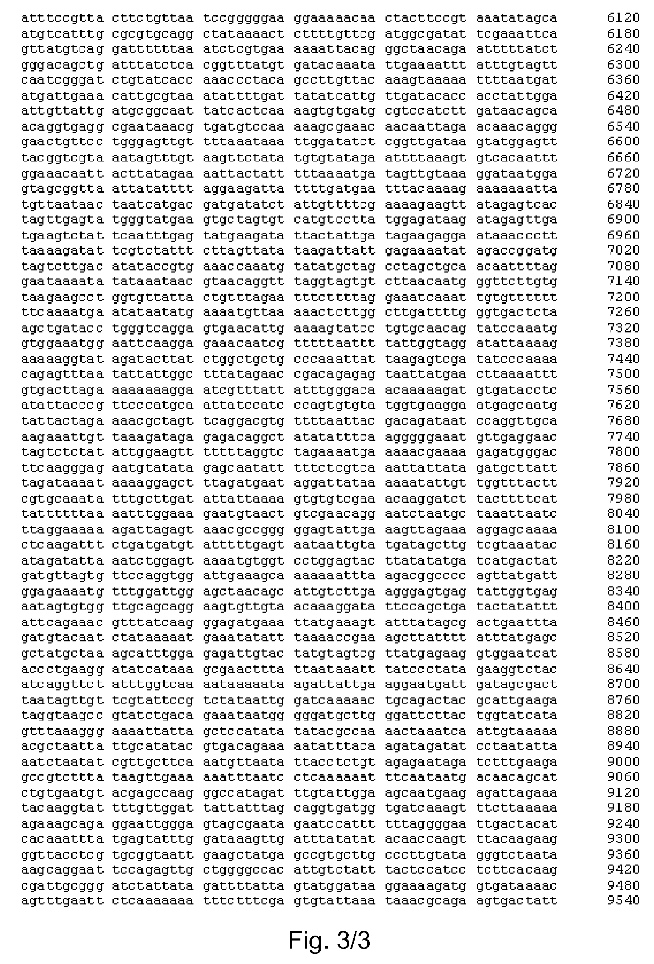

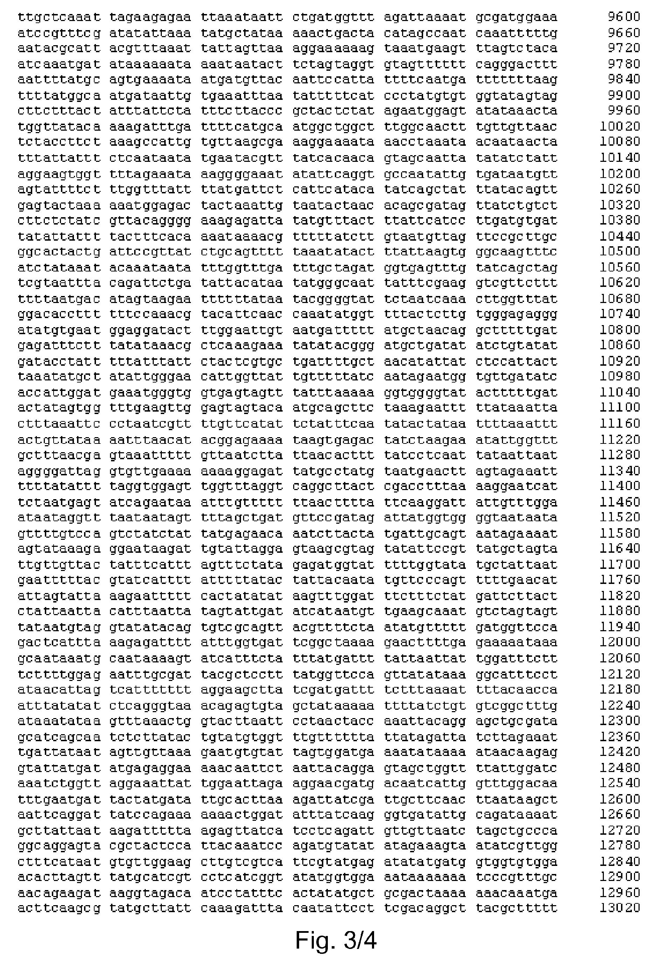

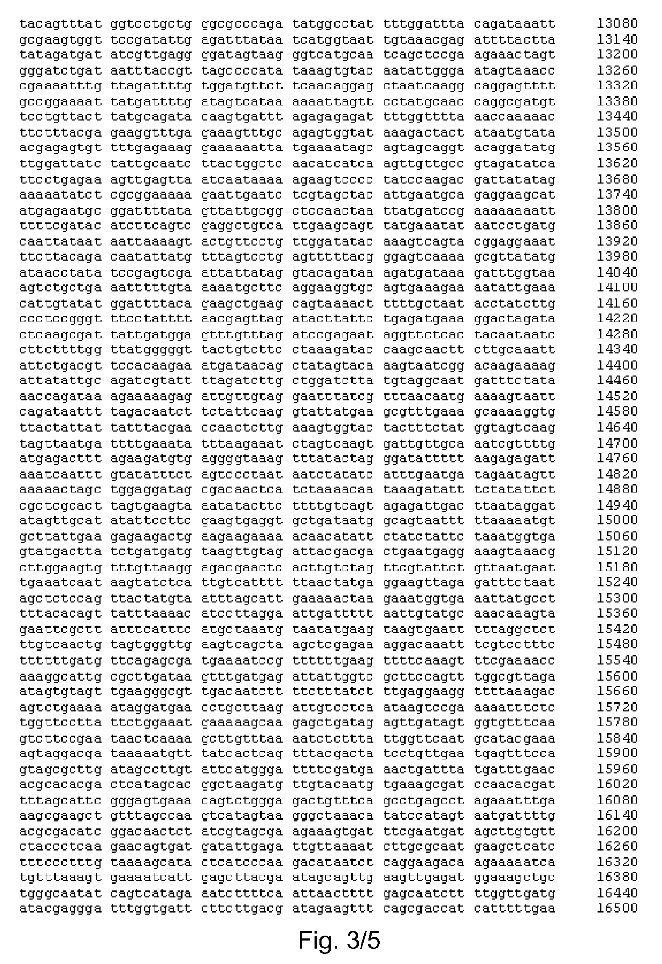

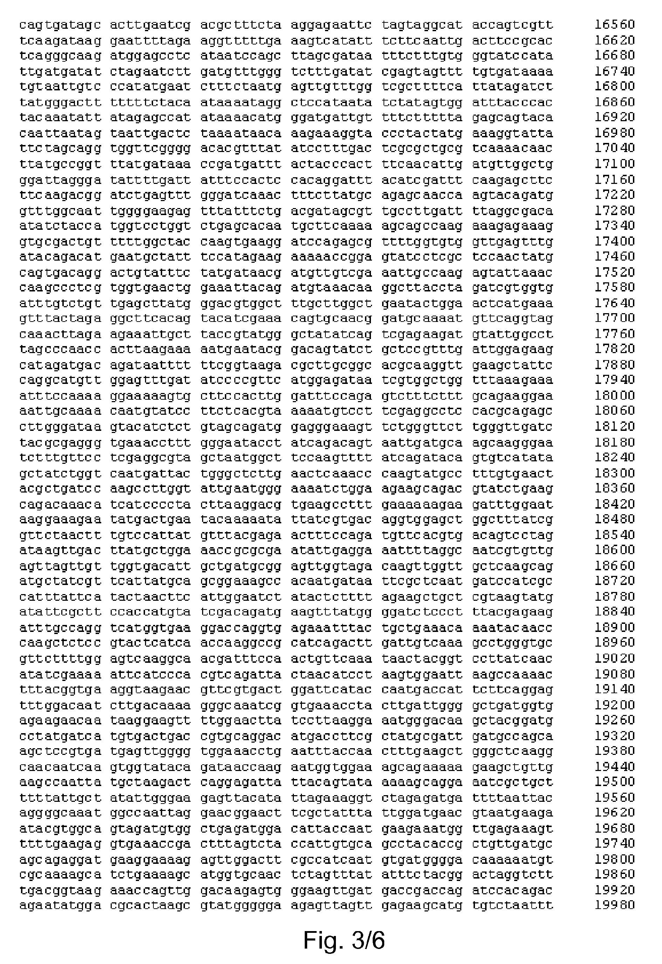

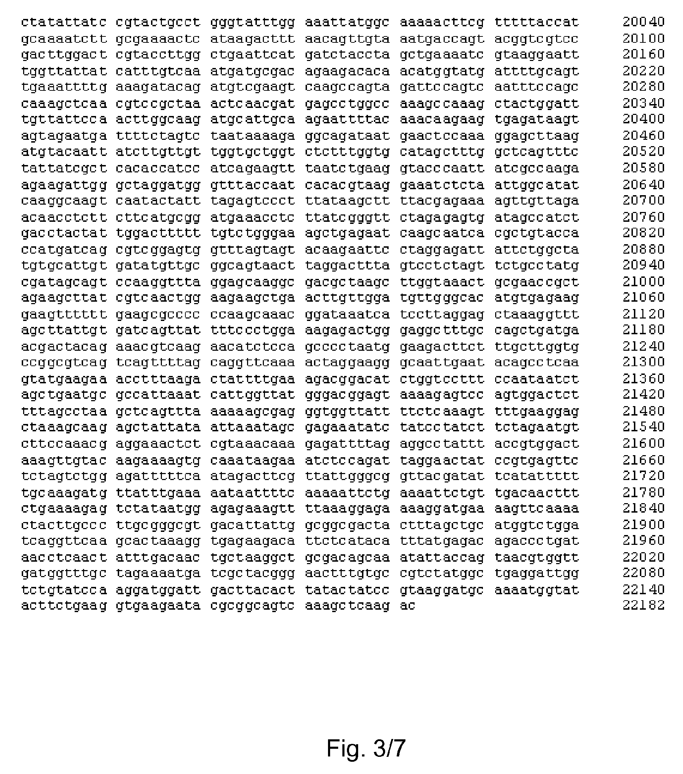

In the following section it is described how the presence of *Streptococcus Pneumoniae* DNA in biological samples and isolates can be evaluated by molecular methods subject of the present invention, preferably using primers and probes with at least one Real-Time Polymerase Chain Reaction (RT) or by methods previously described (MS-PCR). In a preferred embodiment, Primers and probes are designed based on *CpsA gene* (pneumococcal capsular polysaccharide synthesis gene, for serotype 1 the design was based on previously published *Cps gene* locus of SEQ ID. no. 64, Fig. 3). The example described in the section below shows the improvement obtained by the methods subject of the present invention when compared to methods previously described (MS-PCR).

Nonetheless, when a positive result is obtained in samples considered negative by other methods, such as MS-PCR or culture, it might be speculated that this is due to poor specificity of RT-PCR rather than to improved sensitivity. However amplification in RT-PCR at the same time of two different targets from two unrelated genes of *Streptococcus pneumoniae, lytA* (known to be extremely sensitive and specific) [21] and *CpsA* confirms the sample positivity. Realtime PCR in pneumococcal serotyping offers additional chances also in the analysis of pharyngeal carrying. The method subject of the present invention being more sensitive than previously known methods, demonstrate multiple colonization can be found with a frequency that is higher than previously thought with methods known in the art [34].

This aspect has important implications in the study of pneumococcal carriage and serotype replacement. Actually, in carriage studies performed with culture the single colony method is commonly used to determine the serotype by the Quellung reaction. When colonies of multiple morphologies are present, selection by morphology adds some information, but nevertheless both methods underestimate the true rate of multiple carriage [16-18]. Actually, based on the single-colony cultural method, a single serotype per patient can be found and multiple colonization, which is a very frequent condition cannot be revealed [16]. when we are interested in finding all circulating serotypes or when we want to draw conclusions on limited phenomena as initial serotype replacement could be, individuating multiple colonization is mandatory and therefore molecular methods should be used.

RT-PCR is a technique known in the art. Reaction amplification is signalled and, possibly, quantified by use of a fluorescent probe which generates a signal simultaneously with the amplification reaction The nucleic acid sequences resulting from the RT-PCR reaction are DNA sequences. The RT-PCR reaction may be carried out with any equipment or reagent known in the art. In a preferred embodiment of said aspect of the invention wherein the presence or absence of at least a pathogen is detected, use is made of primer and hydrolysis probe sequences, among which, e.g., the TaqMan probes may be used in a PCR amplification cycle reaction known as TaqMan reaction. Said TaqMan probe sequence is a fluorescent probe bonded by means of a nucleic acid sequence to a quencher. The nucleic acid sequence is DNA. The TaqMan probe sequence is hydrolised thanks to the polymerisation of Taq-polymerase (through the action of 3'-5' exonuclease of Taq polymerase). As a consequence, the fluorescent hydrolisis probe or TaqMan probe, without interferences from the quencher, emits fluorescence to signal that polymerization has occurred. TaqMan primers and probes used in the present invention may be built according to methods known in the art to build DNA sequences and methods to bind probes or quenchers thereto. In said aspect of the invention, the RT-PCR reaction may detect the presence of one or more sequences, and consequently, if the reagents are adequately selected, one or more pathogen serotypes at the same time. An advantage of the use of said aspect of the invention, which is the RT-PCR to detect the presence of one or more different sequences, is a reduction of times and/or costs to detect whether and which pathogen serotypes is present in the sample. RT-PCR enables the discrimination among said serotypes with the help of one ore more primer couples (the couple being forward primer and reverse primer for a specific sequence), designed to amplify genes or specific sequences. With the presence of a TaqMan probe, the amplification signals the presence in the sample of extracted nucleic acid. If every nucleic acid sequence is uniquely specific for a single pathogen among those mentioned above, the RT-PCR enables the signalling of their presence.

It is therefore an object of the invention a method for detecting a specific serotype of *S*. *pneumoniae* in a sample, comprising the following steps:
a) extracting *S. pneumoniae* DNA from the sample;
b) incubating the extracted DNA under conditions such as to enable the amplification of at least one serotype specific target region comprised in the *Cps* gene cluster locus of *S. pneumoniae* by real-time PCR using at least SEQ ID NO. 4 and SEQ ID NO. 5 as forward and reverse primers and at least SEQ ID NO. 6 as probe sequence for serotype 3 if present in the sample, and
c) detecting and identifying the amplified product. Preferably, the step b) of the method according to the invention enables the amplification of at least one further serotype specific target region comprised in the *Cps* gene cluster locus of *S. pneumoniae,*
selected from the group of:
- SEQ ID No. 98 for serotype 1;
- SEQ ID No. 100 for serotype 4;
- SEQ ID No. 101 for serotype 5;
- SEQ ID No. 102 for serotype 6A/B;
- SEQ ID No. 103 for serotype 7A/F;
- SEQ ID No. 104 for serotype 8;
- SEQ ID No. 105 for serotype 9V/A;
- SEQ ID No. 106 for serotype 10A/B;
- SEQ ID No. 107 for serotype 12A/B/F;
- SEQ ID No. 108 for serotype 14;
- SEQ ID No. 109 for serotype 15;
- SEQ ID No. 110 for serotype 18B/C;
- SEQ ID No. 111 for serotype 19 B/F;
- SEQ ID No. 112 for serotype 19A;
- SEQ ID No. 113 for serotype 20;
- SEQ ID No. 114 for serotype 22F/A;
- SEQ ID No. 115 for serotype 23F;
- SEQ ID No. 116 for serotype 33 A/F ;
- SEQ ID No. 117 for serotype 35B ;
- SEQ ID No. 118 for serotype 38 ;
- SEQ ID No. 119 for serotype 16F ;
- SEQ ID No. 120 for serotype 7B/7F ;
- SEQ ID No. 121 for serotype 35F (47F) ;
- SEQ ID No. 122 for serotype 17F ;
- SEQ ID No. 123 for serotype 23B ;
- SEQ ID No. 124 for serotype 23A ;
- SEQ ID No. 125 for serotype 29 ;
- SEQ ID No. 126 for serotype 2 ;
- SEQ ID No. 127 for serotype 11 ;
- SEQ ID No. 128 for serotype 21 ;
or
SEQ ID No. 129 for serotype 24A/24F.

In the present invention the sample may be a biological sample, in particular obtained from a subject, it may also be an agricultural, soil, water or any sample.

Preferably the steps of amplification and detection occur by RT-PCR.

It is a further object of the invention a kit for amplifying and detecting at least one serotype specific target region comprised in the *Cps* gene cluster locus of *S. pneumoniae* as indicated above comprising:
- at least one set of primer oligonucleotides and at least one probe oligonucleotide able to specifically amplify and detect at least one target region indicated above wherein said primer oligonucleotides are at least SEQ ID NO. 4 and SEQ ID NO. 5 as forward and reverse primers and said probe is at least SEQ ID NO. 6 as probe sequence for serotype 3 , and
- a positive control.

The set of primers for the positive control amplification may be obtained from lytA gene and may be selected among the set of SEQ ID 1 and SEQ ID 2, or SEQ ID 7 and SEQ ID 8.

In a preferred embodiment the further set of primer oligonucleotides and the further probe oligonucleotide are selected from the group of:
- SEQ ID NO. 1 and 2 as forward and reverse primers and SEQ ID NO. 3 as probe sequence for serotype 1;
- SEQ ID NO. 7 and 8 as forward and reverse primers and SEQ NO. 9 as probe sequence for serotype 4;
- SEQ ID NO. 10 and 11 as forward and reverse primers and SEQ NO. 12 as probe sequence for serotype 5;
- SEQ ID NO. 13 and 14 as forward and reverse primers and SEQ ID NO. 15 as probe sequence for serotype 6A/B;
- SEQ ID NO. 16 and 17 as forward and reverse primers and SEQ NO. 18 as probe sequence for serotype 7F;
- SEQ ID NO. 19 and 20 as forward and reverse primers and SEQ ID NO. 21 as probe sequence for serotype 8;
- SEQ ID NO. 22 and 23 as forward and reverse primers and SEQ NO. 24 as probe sequence for serotype 9V/A;
- SEQ ID NO. 25 and 26 as forward and reverse primers and SEQ NO. 27 as probe sequence for serotype lOAF;
- SEQ ID NO. 28 and 29 as forward and reverse primers and SEQ NO. 30 as probe sequence for serotype 12ABF;
- SEQ ID NO. 31 and 32 as forward and reverse primers and SEQ NO. 33 as probe sequence for serotype 14;
- SEQ ID NO. 34 and 35 as forward and reverse primers and SEQ NO. 36 as probe sequence for serotype 15;
- SEQ ID NO. 37 and 38 as forward and reverse primers and SEQ NO. 39 as probe sequence for serotype 18B/C;
- SEQ ID NO. 40 and 41 as forward and reverse primers and SEQ NO. 42 as probe sequence for serotype 19A;
- SEQ ID NO. 43 and 44 as forward and reverse primers and SEQ NO. 45 as probe sequence for serotype 19 B/F;
- SEQ ID NO. 46 and 47 as forward and reverse primers and SEQ NO. 48 as probe sequence for serotype 20;
- SEQ ID NO. 49 and 50 as forward and reverse primers and SEQ NO. 51 as probe sequence for serotype 22F/A;
- SEQ ID NO. 52 and 53 as forward and reverse primers and SEQ ID NO. 54 as probe sequence for serotype 23F;
- SEQ ID NO. 55 and 56 as forward and reverse primers and SEQ ID NO. 57 as probe sequence for serotype 33 A/F;
- SEQ ID NO. 58 and 59 as forward and reverse primers and SEQ ID NO. 60 as probe sequence for serotype 35B;
- SEQ ID NO. 61 and 62 as forward and reverse primers and SEQ ID NO. 63 as probe sequence for serotype 38;
- SEQ ID NO. 65 and 66 as forward and reverse primers and SEQ ID NO. 67 as probe sequence for serotype 16F;
- SEQ ID NO. 68 and 69 as forward and reverse primers and SEQ ID NO. 70 as probe sequence for serotype 7B/7C;
- SEQ ID NO. 71 and 72 as forward and reverse primers and SEQ ID NO. 73 as probe sequence for serotype 35F (47F);
- SEQ ID NO. 74 and 75 as forward and reverse primers and SEQ ID NO. 76 as probe sequence for serotype 17F;
- SEQ ID NO. 77 and 78 as forward and reverse primers and SEQ ID NO. 79 as probe sequence for serotype 23B;
- SEQ ID NO. 80 and 81 as forward and reverse primers and SEQ ID NO. 82 as probe sequence for serotype 23A;
- SEQ ID NO. 83 and 84 as forward and reverse primer for serotype 29 and SEQ ID NO. 85 as probe sequence for serotype 29;
- SEQ ID NO. 86 and 87 as forward and reverse primers and SEQ ID NO. 88 as probe sequence for serotype 2;
- SEQ ID NO. 89 and 90 as forward and reverse primers and SEQ ID NO. 91 as probe sequence for serotype 11;
- SEQ ID NO. 92 and 93 as forward and reverse primers and SEQ ID NO. 94 as probe sequence for serotype 21;
- SEQ ID NO. 95 and 96 as forward and reverse primers and SEQ ID NO. 97 as probe sequence for serotype 24A/24F.

In a still preferred embodiment the further set of primer oligonucleotides and the further probe oligonucleotide are selected from the group of:
- SEQ ID NO. 1 and 2 as forward and reverse primers and SEQ ID NO. 3 as probe sequence for serotype 1;
- SEQ ID NO. 7 and 8 as forward and reverse primers and SEQ NO. 9 as probe sequence for serotype 4;
- SEQ ID NO. 10 and 11 as forward and reverse primers and SEQ NO. 12 as probe sequence for serotype 5;
- SEQ ID NO. 13 and 14 as forward and reverse primers and SEQ NO. 15 as probe sequence for serotype 6A/B;
- SEQ ID NO. 16 and 17 as forward and reverse primers and SEQ NO. 18 as probe sequence for serotype 7F;
- SEQ ID NO. 19 and 20 as forward and reverse primers and SEQ NO. 21 as probe sequence for serotype 8;
- SEQ ID NO. 22 and 23 as forward and reverse primers and SEQ NO. 24 as probe sequence for serotype 9V/A;
- SEQ ID NO. 31 and 32 as forward and reverse primers and SEQ NO. 33 as probe sequence for serotype 14;
- SEQ ID NO. 34 and 35 as forward and reverse primers and SEQ NO. 36 as probe sequence for serotype 15;
- SEQ ID NO. 37 and 38 as forward and reverse primers and SEQ ID NO. 39 as probe sequence for serotype 18B/C;
- SEQ ID NO. 40 and 41 as forward and reverse primers and SEQ NO. 42 as probe sequence for serotype 19A;
- SEQ ID NO. 43 and 44 as forward and reverse primers and SEQ NO. 45 as probe sequence for serotype 19 B/F;
- SEQ ID NO. 49 and 50 as forward and reverse primers and SEQ NO. 51 as probe sequence for serotype 22F/A;
- SEQ ID NO. 52 and 53 as forward and reverse primers and SEQ NO. 54 as probe sequence for serotype 23F.

In a yet preferred embodiment the set of primer oligonucleotides and the probe oligonucleotides for different serotypes are grouped in one or more reaction environments.

In a preferred embodiment of said aspect of the invention, the nucleic acid sequences are DNA because RNA is labile and does not keep for a long time and Taq DNA polymerase works better with DNA. In a more preferred embodiment, DNA sequences are genes or specific sequences of the serotypes. Still more preferably said genes or specific sequences of the serotypes are from CpsA gene.

Preferably, primer oligonucleotides are grouped in a plurality of reaction environments. Each group may contain 2 primer/probe sets. Any combination of primer/probe sets specific for different serotypes is accepted.

The RT-PCR reaction is carried out for n amplification cycles, preferably more than 40, even more preferably from 43 to 45 cycles, even more preferably 45 cycles. The reliability of the diagnostic outcome from this aspect of the invention is improved based on the number of RT-PCR cycles and 45 is the best value. An amplification of more than 45 cycles does not improve the reliability of the diagnostic outcome. The diagnostic outcome is compared with the threshold cycle (CT). The CT is the cycle wherein the fluorescence signals emitted are neatly measurable and statistically valid relative to the background noise. If at CT a significantly higher fluorescence and statistically meaningful is found, there may be determined, based on the frequency of the fluorescence emitted and on how the TaqMan fluorescent probes have been prepared in the TaqMan probes, which sequence, and therefore which serotype, is present in the sample and which is not. If no spontaneous fluorescence (i.e. different from the background noise) is measured, one may infer that the sequences specific for the serotype are not present in the DNA extracted from the sample taken.

According to said aspect of the invention, if the presence of said genes at the end of said RT-PCR reaction is detected or not detected, it may be concluded that said serotype is or is not present in the sample taken from the host. Embodiments of said aspect of the invention are reported in even greater detail in the example. An advantage of the present method over the methods of the known art is that it allows the use of nucleic acid, preferably DNA extracted from a sample of biological tissue.

The invention will be now illustrated by the following non limiting examples, figures and tables.

### EXAMPLES

### Streptococcus Pneumoniae isolates

Eight bacterial strains of *Streptococcus Pneumoniae* were obtained from ATCC (respectively ATCC 6301 for 1, ATCC 6304 for 4, ATCC 6305 for 5, ATCC 10357 for 19A, ATCC 6323 for 23F, ATCC 6326 for 6B, ATCC 10368 for 9V, ATCC BAA-659 for 6A). 38 well characterized S.pneumoniae isolates from eighteen serotypes (1, 3, 4, 5, 6A, 6B, 7F, 8, 9V, 12F, 14, 15B/C, 18C, 19A, 19F, 22F, 23F,35B) which had been isolated from blood or CSF or throat swab samples and stored in germ bank at the Microbiology Laboratory of the Anna Meyer Children Hospital were used for molecular serotyping.

### Biological samples

Sixty-nine biological samples (27 blood samples, 4 cerebrospinal fluid (CSF) samples, 4 pleural fluids and 34 pharyngeal swabs), found positive for *Streptococcus Pneumoniae* by Realtime PCR on *lytA* gene as previously described [3] and obtained from children with a clinical suspicion of invasive pneumococcal infection were tested for *CpsA* by standard PCR and agarose gel electrophoresis as described in following paragraphs; samples positive for both *lytA* and *CpsA* were included in serotyping analyses both by multiplex-sequential PCR (MS-PCR) and Realtime-PCR.

### Bacterial DNA extraction

Bacterial genomic DNA was extracted from bacterial strains or biological samples using QIAmp DNeasy Blood & Tissue kit (Qiagen, Hilden, Germany) with protocol for blood and body fluids, according to the manufacturer's instructions for extraction of a 200 µL sample.

### Molecular diagnosis di Streptococcus pneumoniae

The presence of *Streptococcus Pneumoniae* DNA in biological samples and isolates was evaluated by molecular methods as previously described [3]. Briefly, primers and probes within the autolysin gene *lytA* [21] were used for RT amplification at a final concentration of 300 nM for primers, and 50 nM for JOE-labeled probes. The cycle threshold (*C_{T}*) value is the PCR cycle number (out of 45) at which the measured fluorescent signal exceeds a calculated background threshold identifying amplification of the target sequence. If no increase in fluorescent signal is observed after 45 cycles, the sample is assumed to be negative. The presence of *Streptococcus Pneumoniae* was furtherly confirmed by the amplification of *CpsA* gene in multiplex sequential PCR as previously described [3-4] and briefly described in the next paragraph.

### PCR serotyping by Multiplex sequential PCR and standard cultural methods

Molecular serotyping by sequential multiplex PCR was performed both on bacterial isolates and directly on clinical samples. All the RT-PCR positive samples were included in the analysis.

Briefly, thirty-one primer couples [3] were grouped into nine multiplex reactions. *CpsA* (pneumococcal capsular polysaccharide synthesis gene) primers were included in all the reaction mix as a confirmatory test [3, 22]. Amplification was performed in a Perkin-Elmer GeneAmp PCR system 2720 (Applied Biosystems, Foster City, CA, USA) under the following conditions: 95°C for 15 minutes followed by 35 amplification cycles of 94°C for 30 seconds, 54°C for 90 seconds, and 72°C for 60 seconds. A final hold was performed at 72°C for 10 minutes. The PCR products were analyzed by gel electrophoresis on 2% NuSieve agarose gels (Cambrex Bio Science, Inc., Rockland, ME) in 1x TAE buffer.

Cultural serotyping was performed using specific pneumococcal antisera on bacterial isolates (Biogenetics Srl, Padova, Italy) following manufacturer instructions.

### PCR serotyping by Realtime PCR

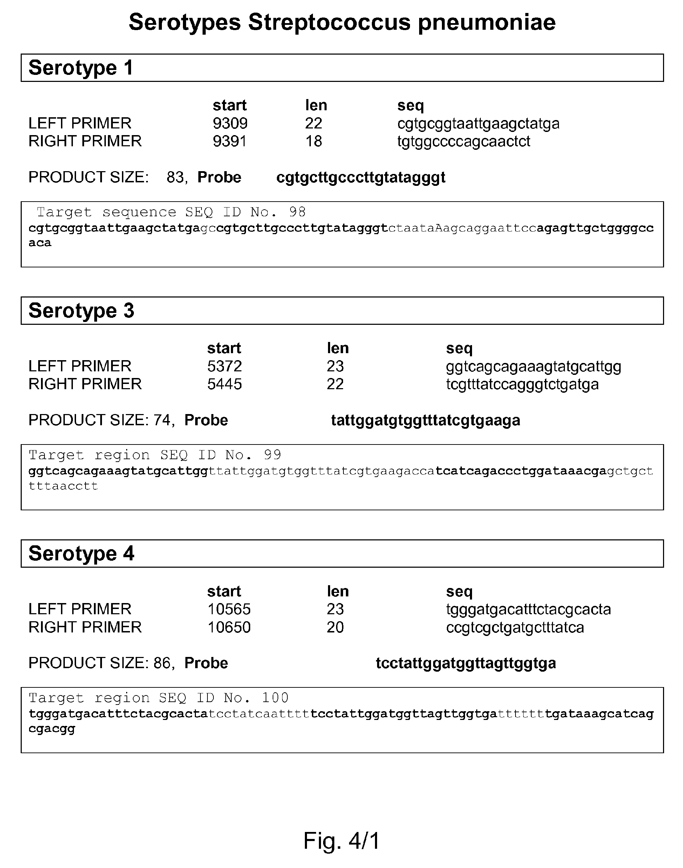

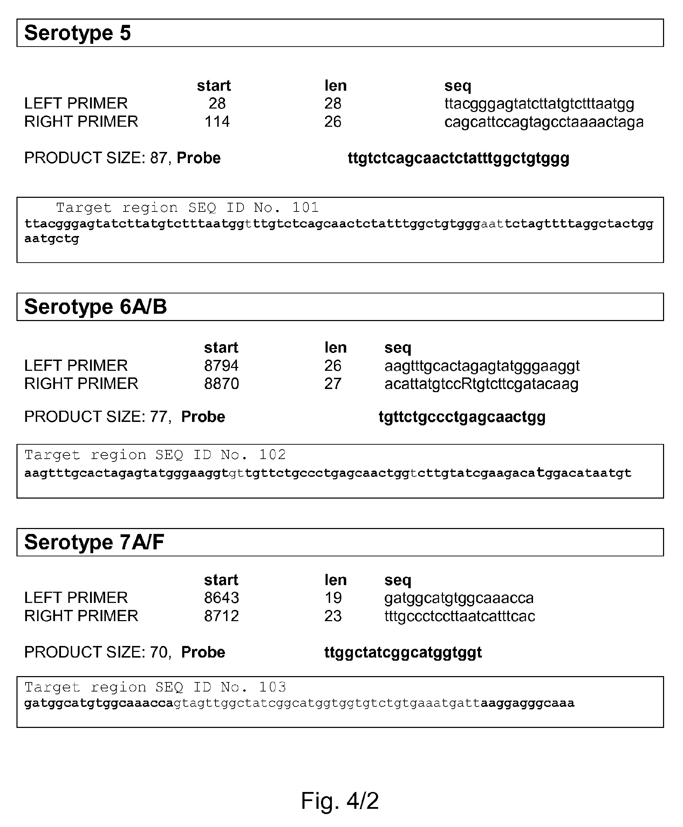

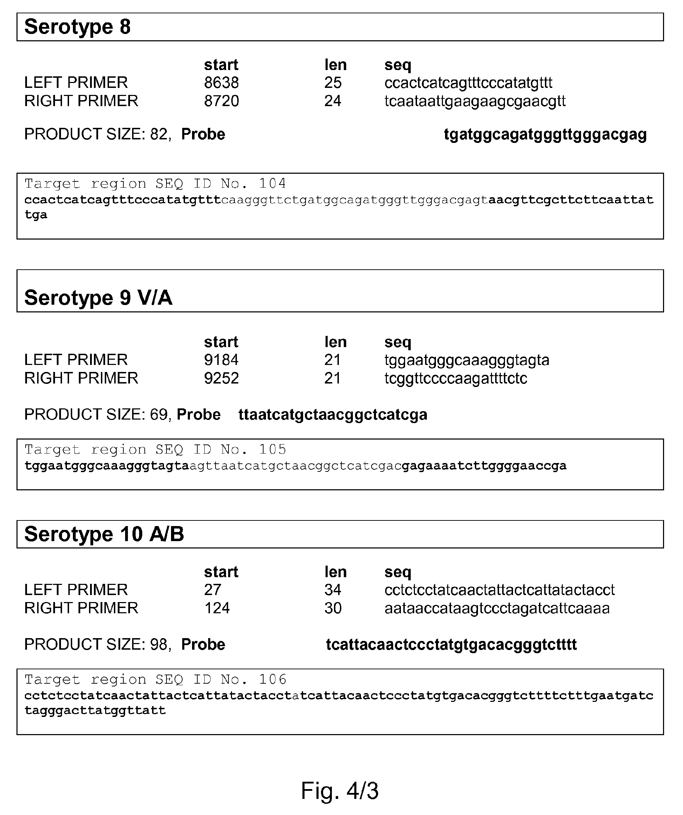

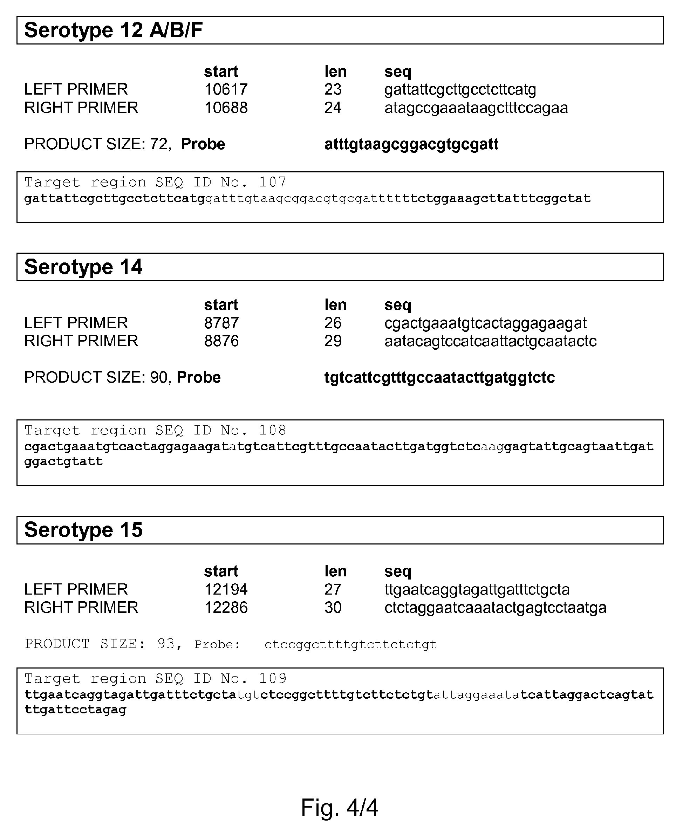

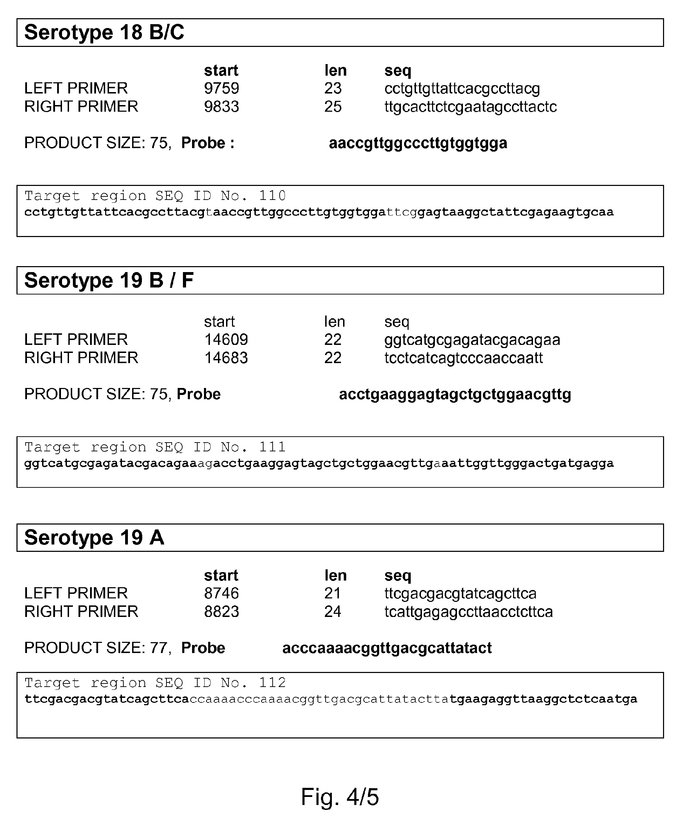

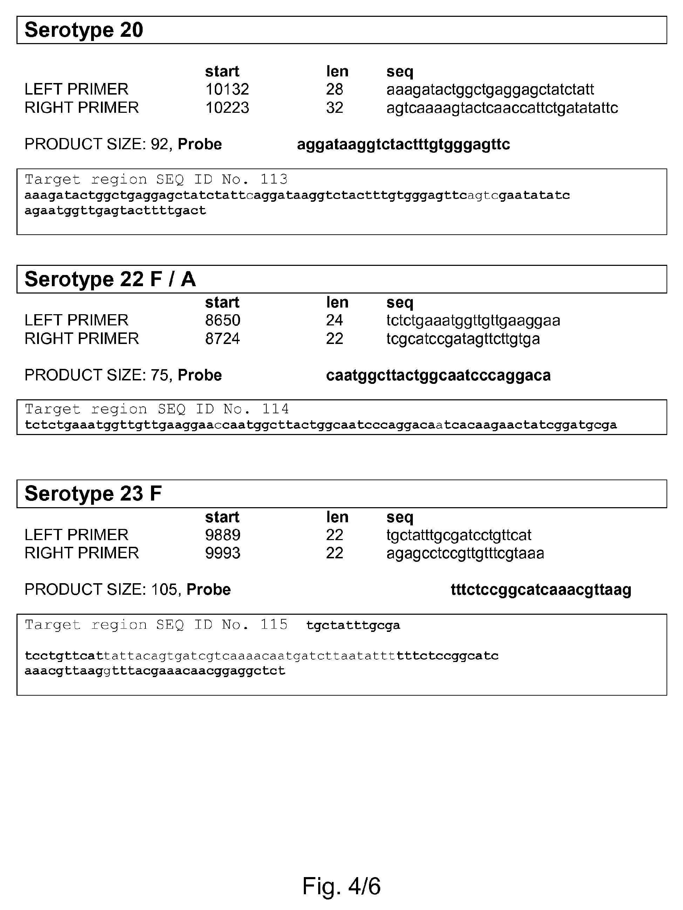

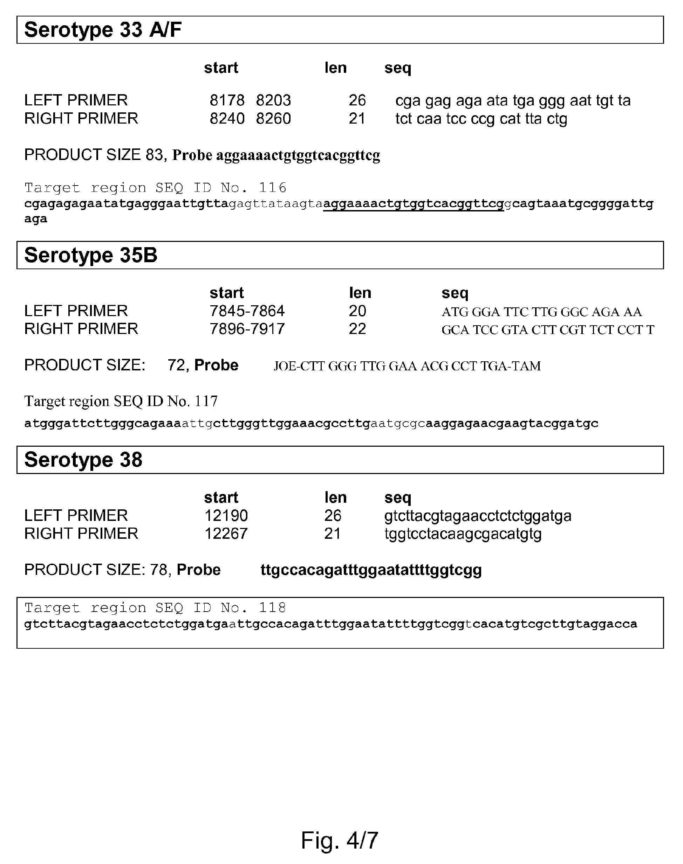

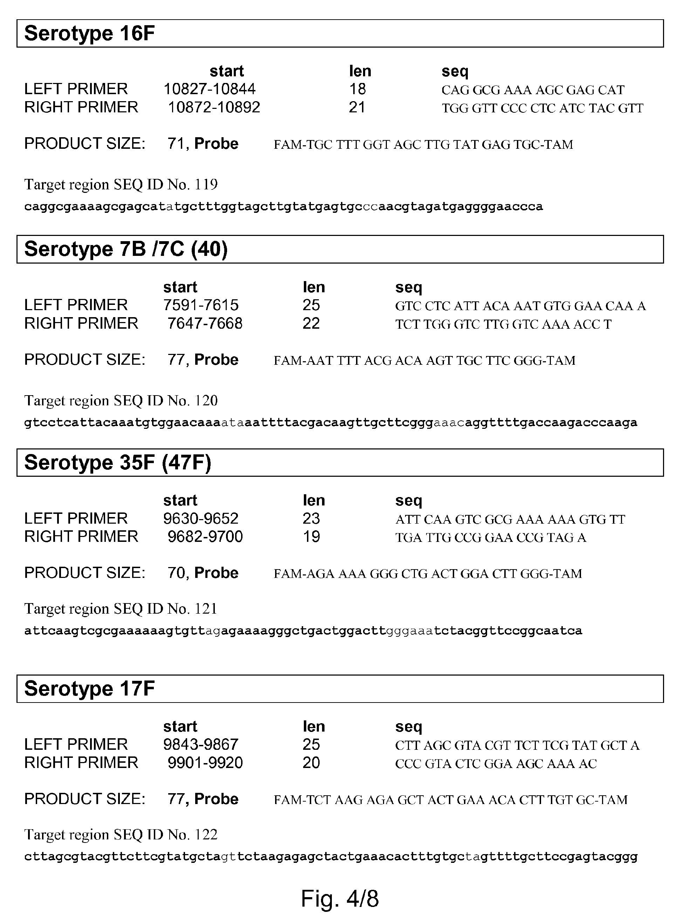

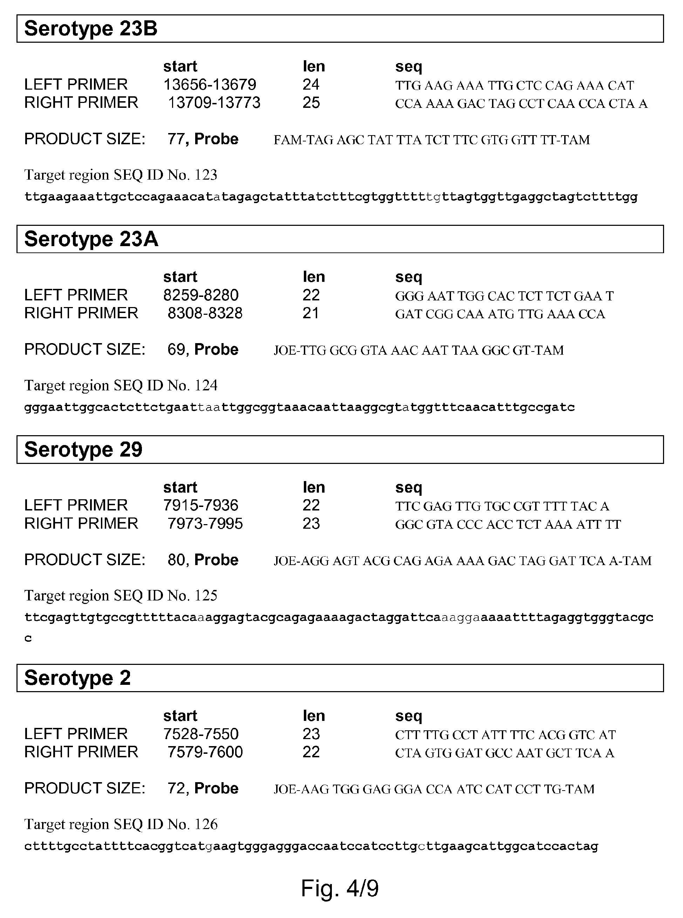

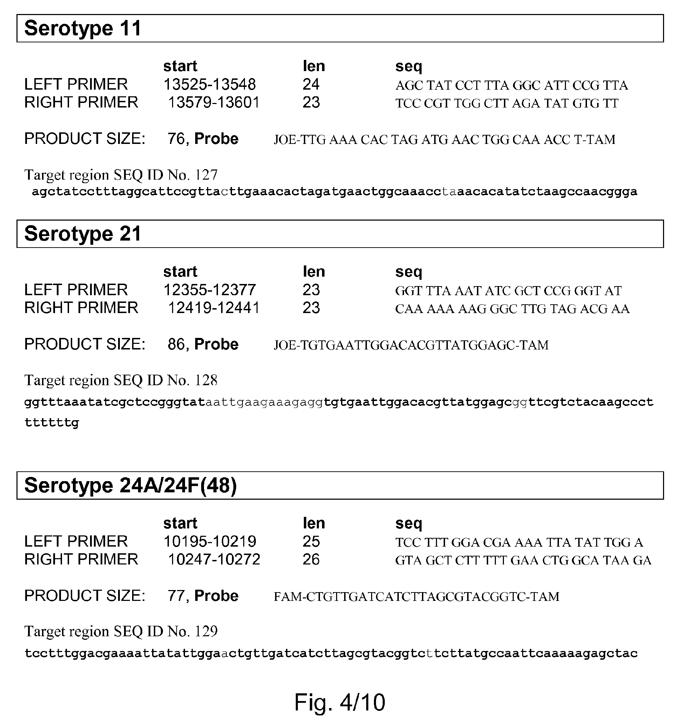

Primers and probes (Tables 1 and 2 and Fig. 4) were designed using the ABI Primer Express Software Package. For serotype 1 the design was based on previously published *Cps gene* locus of SEQ ID. no. 64 (Fig. 3).

**Table 1 - Primer and probe sets for pneumococcal serotyping by Realtime PCR**

| S. pneum. serotype | | Forward primer | | Reverse primer | | Probe |
|---|---|---|---|---|---|---|
| 1 | SEQ ID NO. 1 | cgtgcggtaattgaagctatga | SEQ ID NO. 2 | tgtggccccagcaactct | SEQ ID NO. 3 | JOE-cgtgcttgcccttgtatagggt |
| 3 | SEQ ID NO. 4 | ggtcagcagaaagtatgcattgg | SEQ ID NO. 5 | tcgtttatccagggtctgatga | SEQ ID NO. 6 | JOE-tattggatgtggtttatcgtgaaga |
| 4 | SEQ ID NO. 7 | tgggatgacatttctacgcacta | SEQ ID NO. 8 | ccgtcgctgatgctttatca | SEQ ID NO. 9 | JOE-tcctattggatggttagttggtga |
| 5 | SEQ ID NO. 10 | ttacgggagtatcttatgtctttaatgg | SEQ ID NO. 11 | cagcattccagtagcctaaaactaga | SEQ ID NO. 12 | JOE-ttgtctcagcaactctatttggctgtggg |
| 6A/B | SEQ ID NO. 13 | aagtttgcactagagtatgggaaggt | SEQ ID NO. 14 | acattatgtccRtgtcttcgatacaag | SEQ ID NO. 15 | FAM-tgttctgccctgagcaactgg |
| 7 A/F | SEQ ID NO. 16 | gatggcatgtggcaaacca | SEQ ID NO. 17 | tttgccctccttaatcatttcac | SEQ ID NO. 18 | JOE-ttggctatcggcatggtggt |
| 8 | SEQ ID NO. 19 | ccactcatcagtttcccatatgttt | SEQ ID NO. 20 | tcaataattgaagaagcgaacgtt | SEQ ID NO. 21 | FAM-tgatggcagatgggttgggacgag |
| 9V/A | SEQ ID NO. 22 | tggaatgggcaaagggtagta | SEQ ID NO. 23 | tcggttccccaagattttctc | SEQ ID NO. 24 | FAM-ttaatcatgctaacggctcatcga |
| 10A/B | SEQ ID NO. 25 | cctctcctatcaactattactcattatactacct | SEQ ID NO. 26 | Aataaccataagtccctagatcattcaaag | SEQ ID NO. 27 | FAM-tcattacaactccctatgtgacacgggtctttt |
| 12 A/B/F | SEQ ID NO. 28 | gattattcgcttgcctcttcatg | SEQ ID NO. 29 | atagccgaaataagctttccagaa | SEQ ID NO. 30 | FAM-atttgtaagcggacgtgcgatt |
| 14 | SEQ ID NO. 31 | cgactgaaatgtcactaggagaagat | SEQ ID NO. 32 | Aatacagtccatcaattactgcaatactc | SEQ ID NO. 33 | FAM-tgtcattcgtttgccaatacttgatggtctc |
| 15 | SEQ ID NO. 34 | ttgaatcaggtagattgatttctgcta | SEQ ID NO. 35 | ctctaggaatcaaatactgagtcctaatga | SEQ ID NO. 36 | FAM- ctccggcttttgtcttctctgt |
| 18B/C | SEQ ID NO. 37 | cctgttgttattcacgccttacg | SEQ ID NO. 38 | ttgcacttctcgaatagccttactc | SEQ ID NO. 39 | FAM-aaccgttggcccttgtggtgga |
| 19A | SEQ ID NO. 40 | ttcgacgacgtatcagcttca | SEQ ID NO. 41 | tcattgagagccttaacctcttca | SEQ ID NO. 42 | JOE-acccaaaacggttgacgcattatact |
| 19 B/F | SEQ ID NO. 43 | ggtcatgcgagatacgacagaa | SEQ ID NO. 44 | tcctcatcagtcccaaccaatt | SEQ ID NO. 45 | FAM-acctgaaggagtagctgctggaacgttg |
| 20 | SEQ ID NO. 46 | aaagatactggctgaggagctatctatt | SEQ ID NO. 47 | Agtcaaaagtactcaaccattctgatatattc | SEQ ID NO. 48 | FAM-aggataaggtctactttgtgggagttc |
| 22 A/F | SEQ ID NO. 49 | tctctgaaatggttgttgaaggaa | SEQ ID NO. 50 | tcgcatccgatagttcttgtga | SEQ ID NO. 51 | FAM-caatggcttactggcaatcccaggaca |
| 23F | SEQ ID NO. 52 | tgctatttgcgatcctgttcat | SEQ ID NO. 53 | agagcctccgttgtttcgtaaa | SEQ ID NO. 54 | FAM-tttctccggcatcaaacgttaag |
| 33 A/F | SEQ ID NO. 55 | cgagagagaatatgagggaattgtta | SEQ ID NO. 56 | tctcaatccccgcatttactg | SEQ ID NO. 57 | FAM-aggaaaactgtggtcacggttcg |
| 35B | SEQ ID NO. 58 | atg gga ttc ttg ggc aga aa | SEQ ID NO. 59 | gca tcc gta ctt cgt tct cct t | SEQ ID NO. 60 | JOE-ctt ggg ttg gaa acg cct tga |
| 38 | SEQ ID NO. 61 | gtcttacgtagaacctctctggatga | SEQ ID NO. 62 | tggtcctacaagcgacatgtg | SEQ ID NO. 63 | FAM-ttgccacagatttggaatattttggtcgg |

**Table 2 - Primer and probe sets for pneumococcal serotyping by Realtime PCR**

| Serotype | | Primer forward | | Primer reverse | | Probe |
|---|---|---|---|---|---|---|
| | | | | | | |
| 16F | SEQ ID No. 65 | CAG GCG AAA AGC GAG CAT | SEQ ID No. 66 | TGG GTT CCC CTC ATC TAC GTT | SEQ ID No. 67 | FAM-TGC TTT GGT AGC TTG TAT GAG TGC-TAM |
| 7B /7C (40) | SEQ ID No. 68 | GTC CTC ATT ACA AAT GTG GAA CAA A | SEQ ID No. 69 | TCT TGG GTC TTG GTC AAA ACC T | SEQ ID No. 70 | FAM-AAT TTT ACG ACA AGT TGC TTC GGG-TAM |
| 35F (47F) | SEQ ID No. 71 | ATT CAA GTC GCG AAA AAA GTG TT | SEQ ID No. 72 | TGA TTG CCG GAA CCG TAG A | SEQ ID No. 73 | FAM-AGA AAA GGG CTG ACT GGA CTT GGG-TAM |
| 17F | SEQ ID No. 74 | CTT AGC GTA CGT TCT TCG TAT GCT A | SEQ ID No. 75 | CCC GTA CTC GGA AGC AAA AC | SEQ ID No. 76 | FAM-TCT AAG AGA GCT ACT GAA ACA CTT TGT GC-TAM |
| 23B | SEQ ID No. 77 | TTG AAG AAA TTG CTC CAG AAA CAT | SEQ ID No. 78 | CCA AAA GAC TAG CCT CAA CCA CTA A | SEQ ID No. 79 | FAM-TAG AGC TAT TTA TCT TTC GTG GTT TT-TAM |
| 23A | SEQ ID No. 80 | GGG AAT TGG CAC TCT TCT GAA T | SEQ ID No. 81 | GAT CGG CAA ATG TTG AAA CCA | SEQ ID No. 82 | JOE-TTG GCG GTA AAC AAT TAA GGC GT-TAM |
| 29 | SEQ ID No. 83 | TTC GAG TTG TGC CGT TTT TAC A | SEQ ID No. 84 | GGC GTA CCC ACC TCT AAA ATT TT | SEQ ID No. 85 | JOE-AGG AGT ACG CAG AGA AAA GAC TAG GAT TCA A-TAM |
| 2 | SEQ ID No. 86 | CTT TTG CCT ATT TTC ACG GTC AT | SEQ ID No. 87 | CTA GTG GAT GCC AAT GCT TCA A | SEQ ID No. 88 | JOE-AAG TGG GAG GGA CCA ATC CAT CCT TG-TAM |
| 11 | SEQ ID No. 89 | AGC TAT CCT TTA GGC ATT CCG TTA | SEQ ID No. 90 | TCC CGT TGG CTT AGA TAT GTG TT | SEQ ID No. 91 | JOE-TTG AAA CAC TAG ATG AAC TGG CAA ACC T-TAM |
| 21 | SEQ ID No. 92 | GGTTTAAATATCGCT CCGGGTAT | SEQ ID No. 93 | CAAAAAAAGGCT TGTAGACGAA | SEQ ID No. 94 | JOE- TGTGAATTGGACACGTTATGGAGC- TAM |
| 24A/24F(48) | SEQ ID No. 95 | TCCTTTGGACGAAA ATTATATTGGA | SEQ ID No. 96 | GTAGCTCTTTTGA ACTGGCATAAGA | SEQ ID No. 97 | FAM- CTGTTGATCATCTTAGCGTACGGTC- TAM |

Fig. 4 shows a list of amplified regions, specific primers and probe for each serotype of S. *pneumoniae.*

The RT amplification was performed in 25 µL reaction volumes containing 2x TaqMan Universal Master Mix (Applied Biosystem, Foster City, CA, USA); primers and JOE labeled probes were used at a concentration of 400nM; FAM labeled probes at a concentration of 200 nM. Six µl of DNA extract were used for each reaction. All reactions were performed in triplicate. A negative control (no-template) and a positive control were included in every run. DNA was amplified in an ABI 7500 sequence detection system (Applied Biosystem, Foster City, CA, USA) using, for all the primers couples, the same cycling parameters as follows: 50° for 2 min for UNG digestion, 95°C for 10 min followed by 45 cycles of a two-stage temperature profile of 95°C for 15 sec and 60°C for 1 min.

As for molecular diagnosis of IPD (invasive pneumococcal didease) by realtime PCR, if no increase in fluorescent signal was observed after 45 cycles, the sample was assumed to be negative and was reported as non-typeable.

### Pneumococcal serotype classification and correlation with the existing conjugate vaccine

As previously described [23], pneumococcal serotypes were classified as "PCV-7 serotypes" if they were included in the conjugate vaccine (4,6B,9V,14,18C,19F or 23F). They were classified as "PCV-7 related serotypes" if they were from the same serogroups and were assumed or known to be cross-reactive with PCV7 serotypes (6A,9A,9L,9N,18A,18B,18F,19B,19C,23A,23B); all the other, including 19A, known to be non cross-reactive with 19F, were classified as "non-vaccine serotypes" [23].

### Statistical analysis

Data were evaluated to determine how many samples were positive by each test, as well as those positive by two tests and those positive by only one test. Agreement between the two tests was assessed by Cohen's kappa statistic, with values of 0.00 to 0.20 indicating poor agreement, 0.21 to 0.40 indicating fair agreement, 0.41 to 0.60 indicating moderate agreement, 0.61 to 0.80 indicating good agreement, and 0.81 to 1.00 indicating excellent agreement. Marginal homogeneity of the two tests was assessed by McNemar's test. Results were expressed as mean levels and standard deviations. All continuous variables were expressed as mean ± SD. Fisher exact test and Pearson's chi-square test were used when appropriate.

### External Quality Control

An External Quality Control for Real-time PCR and standard PCR is regularly carried out with excellent results in the Laboratory of Immunology where all the molecular test were performed.

### RESULTS

During the set up of the method subject of the present invention, only 15 primer/probe set, representing the most frequent *Streptococcus Pneumoniae* serotypes in western world, were initially included. With such group of primer/probe it was possible to identify and serotype 92% of IPD (invasive pneumococcal disease) cases. A group of 6 additional primer/probe was then added giving a 4% increase in serotype identification. Therefore, it was found that in a preferred embodiment, the present invention uses a panel of primer/probe including 21 serotypes that allows to achieve, at present, pneumococcal serotyping in over 94% cases in Italy and likely in most western countries where the distribution is similar. In details, using the primer/probe sets described in the present invention the highest efficiency and the best cost/benefit ratio are obtained. In fact, the optimized combination gives the highest frequency of serotyping results with the less expense. Moreover, the method of the present invention allows to include primers/probe for additional serotypes, therefore it can be adapted to possible epidemiological changes and serotype shifts over the years and in different parts of the world.

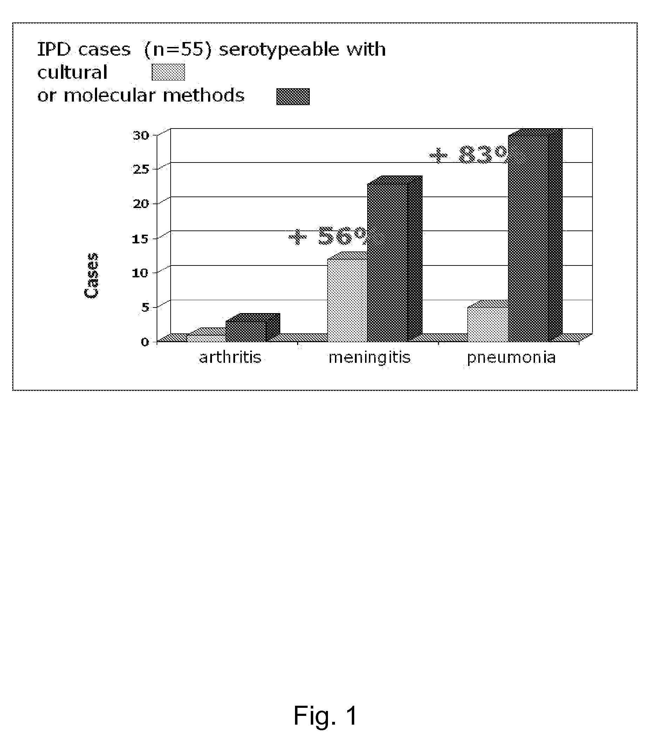

The amplified sequences detected with the primer/probe sets here described can be considered as molecular markers for invasive pneumococcal infections due to different serotypes. Each amplified sequence is to be considered, therefore as a specific marker for a unique infection. In conclusion, using the present invention's method, based on Realtime PCR in a clinical setting, it is possible to achieve diagnosis and serotyping of invasive pneumococcal infection in cases for which the diagnosis was not possible with other methods used before. As from examples given in the section below, 83% pneumonia cases diagnosed and serotyped by Realtime PCR would not have been serotyped using only cultural methods, similarly 56% meningitis would not have been serotyped (Fig.1).

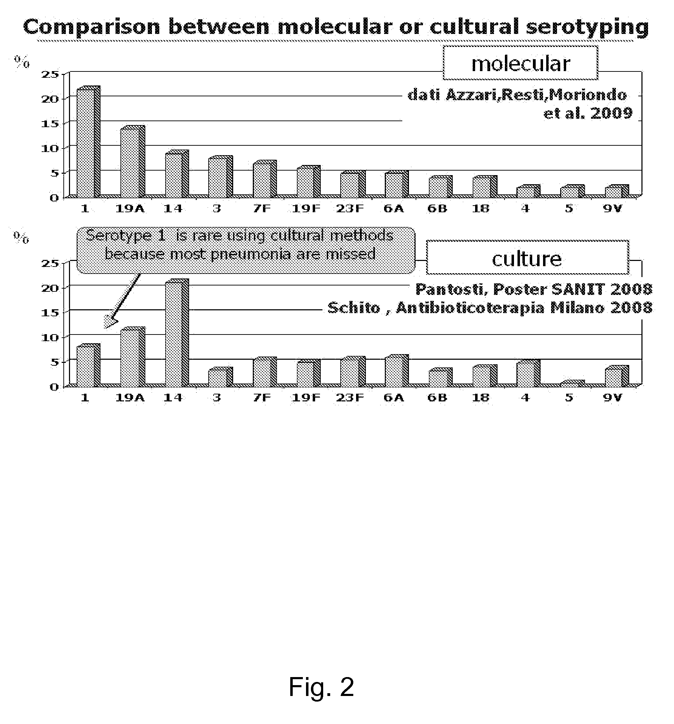

Moreover, serotype distribution as evidenced by Realtime PCR appears different from what is previously known. Since present cultural methods fail to detect most cases of bacteremic pneumococcal pneumonia, the frequency of serotype 1 detected using such method is significantly lower than that detected by Realtime PCR (Fig.2).

### Molecular diagnosis of Streptococcus pneumoniae

### Isolates

All the pneumococcal isolates (either obtained from ATCC or obtained from the microbiology lab) resulted positive with both MS-PCR (CpsA gene) and RT PCR (lytA gene).

### Biological samples

Positivity for both *CpsA* in MS-PCR and *lytA* in RT-PCR was found in 67/69 (97.1%) samples. The two samples (blood) negative for *CpsA* in MS-PCR had a RT-PCR *C_{T}* respectively of 38 and 39. Only the 67 samples positive for both *lytA* gene in RT-PCR and *CpsA* gene in MS-PCR were included in the study.

### Serotyping of Streptococcus Pneumoniae isolates

The eight isolates obtained from ATCC were serotyped by both molecular methods and were consistent with ATCC serotype.

All the 38 *Streptococcus Pneumoniae* isolates serotyped by the two molecular methods showed a complete concordance with serologic typing (serotype 3:7 isolates; serotypes 1, 4, 5, 6A: 3 isolates/each; serotypes 6B,7F, 8, 9V, 12F, 14: 2 isolates/each; serotypes 15B/C, 18C, 19A, 19F, 22F, 23F, 35B 1 isolate/each). No cross-reaction between serotypes was found for any primer/probe set.

### Serotyping of Streptococcus Pneumoniae from biological samples

Among the 67 biological samples found positive for Streptococcus pneumoniae, 43/67 (64.2%; 16/25 blood samples; 4/4 CSF; 4/4 pleural fluid, 19/34 swabs) could be serotyped by MS-PCR, while 61/67 (23/25 whole blood, 4/4 CSF, 4/4 pleural fluid, 30/34 pharyngeal swabs 91.0 %, p=0.001) could be serotyped by RT-PCR (K cohen:0.3; McNemars's p<0.001). The 21 biological samples that could be serotyped only by RT-PCR (7/25 blood samples, 12/34 pharyngeal swabs) had a *C_{T}*(threshold cycle) ≥30 (median 34.5; SD 2.4; range 30-38 with no difference between blood samples and swabs). All CSF and pleural fluid samples could be serotyped with both methods. One sample (pharyngeal swab) that could be serotyped only by MS-PCR was a serotypes 6C/D, primers of which were not available for RT-PCR. Five samples (2 blood samples, 3 pharyngeal swabs) could not be serotyped with any method.

When only samples obtained from otherwise sterile fluids (blood, CSF, pleural fluid) were analysed, MS-PCR allowed serotyping in 24/33 (72.7%) samples (16 WB, 4 CSF, 4 PF obtained from 25 patients) and RT-PCR in 31/33 (93.9%) samples (23 WB, 4 CSF, 4PF) (p=0.047; 95% CL 0.03-0.98). Serotypes found with the two molecular methods are shown in Table 3.

**Table 3- Comparison between Multiplex sequential PCR (MS-PCR) and Realtime PCR (RT-PCR) in evaluating serotype distribution in sterile fluids from 25 patients with pneumonia (P) or meningitis (M) .**

| | **Disease** | **MS-PCR** | **RT-PCR** |
|---|---|---|---|
| 1 | P | 19A | 19A |
| 2 | P | 19A | 19A |
| 3 | P | 1 | 1 |
| 4 | S | 22 A/F | 22 A/F |
| 5 | P | 1 | 1 |
| 6 | M | NT | NT |
| 7 | M | NT | NT |
| 8 | M | NT | 18 |
| 9 | M | 19A | 19A |
| 10 | P | NT | 19F/B |
| 11 | P | NT | 19A |
| 12 | M | 7 A/F | 7 A/F |
| 13 | P | NT | 1 |
| 14 | P | NT | 1 |
| 15 | P | NT | 1 |
| 16 | P | NT | 19A |
| 17 | M | 23F | 23F |
| 18 | M | 23F | 23F |
| 19 | P | 6A | 6A |
| 20 | M | 14 | 14 |
| 21 | M | 6B | 6B |
| 22 | M | 8 | 8 |
| 23 | P | 1.9A | 19A |
| 24 | M | 10 A/B | 10 A/B |
| 25 | M | 23F | 23F |

Multiple colonization by different pneumococcal serotypes was demonstrated in 20/30 (66.7%) pharyngeal swabs typed by RT-PCR, and in 3/19 (15.8%) swabs typed by MS-PCR (p=0.002; 95%CL=2.02-44.13). Realtime PCR demonstrated 1 single serotype in 10/30 (33.3%), 2 different serotypes in 13/30 (43.3%), 3 different serotypes in 6/30 (20.0%) and 4 different serotypes in 1/30 (3.3%); MS-PCR demonstrated 2 different serotypes in 3/19 (15.8%), while in 16/19 samples (84.2%) only one serotype was found (Table 4).

**Table 4 - Multiple pharyngeal colonization shown by Multiplex-sequential PCR (MS) or Real-time PCR (RT) in 34 pharyngeal swabs**

| Sample ID | Method | Scrotypes identified | | | |
|---|---|---|---|---|---|
| | | I serotype | II serotype | III serotype | IV serotype |
| 1 | MS | 22 A/F | | | |
| | RT | 22A/F | 19A | 19F | |
| 2 | MS | NT | | | |
| | RT | NT | | | |
| 3 | MS | 19F | | | |
| | RT | 19F | 5 | | |
| 4 | MS | NT | | | |
| | RT | 4 | 5 | 19F/B | |
| 5 | MS | 6C/D | | | |
| | RT | NT | | | |
| 6 | MS | 5 | | | |
| | RT | 5 | 6A/B | | |
| 7 | MS | 20 | | | |
| | RT | 20 | | | |
| 8 | MS | NT | | | |
| | RT | NT | | | |
| 9 | MS | 9V/A | | | |
| | RT | 9V/A | 5 | | |
| 10 | MS | 14 | | | |
| | RT | 14 | | | |
| 11 | MS | 5 | | | |
| | RT | 5 | PV/A | 19F/B | |
| 12 | MS | NT | | | |
| | RT | 1 | 19A | | |
| 13 | MS | 22A/F | | | |
| | RT | 22A/F | 19A | | |
| 14 | MS | NT | | | |
| | RT | 5 | 19F/B | | |
| 15 | MS | 5 | | | |
| | RT | 5 | 6A/B | | |
| 16 | MS | 5 | | | |
| | RT | 5 | 4 | | |
| 17 | MS | 18 | | | |
| | RT | 18 | 19F/B | 19A | |
| 18 | MS | NT | | | |
| | RT | 1 | 19F/B | | |
| 19 | MS | 1 | | | |
| | RT | 1 | | | |
| 20 | MS | 3 | | | |
| | RT | 3 | 5 | 19F/B | |
| 21 | MS | NT | | | |
| | RT | 1 | | | |
| 22 | MS | NT | | | |
| | RT | 19F/B | | | |
| 23 | MS | NT | | | |
| | RT | 5 | 3 | | |
| 24 | MS | NT | | | |
| | RT | 19F/B | | | |
| 25 | MS | NT | | | |
| | RT | 19F/B | | | |
| 26 | MS | 20 | 5 | | |
| | RT | 20 | 5 | | |
| 27 | MS | NT | | | |
| | RT | NT | | | |
| 28 | MS | 3 | 5 | | |
| | RT | 3 | 5 | | |
| 29 | MS | 19F/B | | | |
| | RT | 19F/B | 18 | | |
| 30 | MS | NT | | | |
| | RT | 1 | | | |
| 31 | MS | 5 | | | |
| | RT | 5 | 7A/F | 18 | 19F/B |
| 32 | MS | 23F | 18 | | |
| | RT | 23F | 18 | 14 | |
| 33 | MS | NT | | | |
| | RT | 19F/B | | | |
| 34 | MS | NT | | | |
| | RT | 18 | | | |
| Total serotypes in MS-PCR | | 22 | | | |
| Mean value p er pharyngeal swab in MS-PCR (range) | | 1.2 serotypes (1-2) | | | |
| Total serotypes in RT-PCR | | 58 | | | |
| Mean value per pharyngeal swab In RT-PCR (range) | | 1.9 serotypes (range 1-4) | | | |

The mean number of serotypes found in each nasopharyngeal swab was 1.2 (range 1-2) for MS-PCR and 1.9 (range 1-4) for RT-PCR (p=0.0006; 95% CL= 025-0.75). Multiple colonization was never found in blood or CSF or pleural fluid.

Multiplex sequential PCR allows pneumococcal serotyping in about two/third cases (64.2%) when used directly on clinical samples (blood, CSF, pleural fluid or nasopharingeal swabs) while Realtime PCR allows pneumococcal serotyping in over 90% cases using a panel of 15 primer/probe sets, with a good concordance between the two tests. However infections due to different serotypes could be diagnosed using additional primer/probe sets. This flexibility is important to make the method suitable all over the world. A starting panel of 30 or more serotypes could be considered, but it would not be economical; in fact a panel of 15 primer/probe sets cover over 90% serotypes while a panel of 30 primer/probe sets adds little more than an additional 5%. However, while in Italy and in other western countries the 15-serotype-panel used in the present research is very efficient in pneumococcal serotyping, individuating with low cost the serotypes in over 90% cases, other serotypes could be more frequent in other parts of the world [29] so requiring different primer/probe sets. Moreover distribution of pneumococcal serotypes may change over years, both as a consequence of immune pressure [30] and of secular trend [31-33]. The present techniques allows to include other primer/probe sets whenever needed without changing laboratory protocols; other primer/probe sets can be easily added or can substitute one or more of the primers included in the present invention Other primer/probe sets can be included in a formulation of the test suitable for other countries in the world.

### EXAMPLE 2: Rare serotypes

As described, the method is endowed with high flexibility, and new serotypes can be added to the test when particular conditions are verified, for instance the primers and probes below are discovered and made available (new serotypes are found in a given geographical area where they were absent before); the frequency of some serotypes, previously considered too low and so irrelevant, becomes important in the epidemiologic context. This happens, for example, when a serotype frequency is found higher than 2%.

With the aim to create diagnostic panels for different regions in the world with respect to a particular pneumococcal epidemiology, a further panel was created and tested. Such panel contains the rarest serotypes in western and industrialized areas, but more frequent in the Southern parts of the world (see Table 2).

The Serotype geographical distribution of table 2 is as follows:
16F Africa, America Latina, USA Europa
7B /7C (40) America Latina, USA
35F (47F) Africa, America Latina, USA
17F USA America Latina Africa
23B USA America Latina Africa
23A USA America Latina Africa Europa
29 USA
2 USA America Latina Africa
11 USA America Latina Africa Europa
35B Africa, America Latina, USA
21 USA America Latina Africa
24A/24F(48) USA America Latina Africa

### REFERENCES

1.Corless CE, et al., J Clin Microbiol 2001; 39: 1553-1558.
2.Pai R., et al., J Clin Microbiol 2006, 44, 124-31
3.Azzari C, et al., J Med Microbiol 2008, 57:1205-1212.
4.Resti M, et al., Clin Therapeutics, 2009, 31: 1266-73.
5.Tarallo L, et al., Vaccine. 2006;24(47-48):6938-43
6.Bridy-Pappas AE, et al., Pharmacotherapy 2005;25:1193-212.
7.World Health Organization (WHO). Wkly Epidemiol Rec 2007; 82:93-104
8.Garau J, et al., Clin Infect Dis. 2007;45(1):52-4.
9.Arai, S., et al., Microbiol Immunol, 2001, 45, 159-162.
10.Lalitha, M. K., et al., J Clin Microbiol 1999 37, 263-265.
11.Barker, J. H. et al., J Clin Microbiol 199, 37, 4039-4041.
12.Heidelberger, M. (1983). Infect Immun 41, 1234-1244.
13.Henrichsen, J. (1999).. Am J Med 107, 50S-54S.
14.Kumar, A., Mariappuram, J. & Kim, C. H. (1985). Diagn Microbiol Infect Dis 3, 509-514.
15. Whitney CG, et al. N Engl J Med 2003;348:1737-46.
16. Charalambous BM, et al., J Clin Microbiol. 2008 May 21.
17.Hare KM, et al., Pediatr Infect Dis J. 2008;27(2):178-80.
18.Azzari C, et al., Clin Infect Dis, 2008; 47:997-999.
19.Heid CA, et al., Genome Res. 1996 Oct;6(10):986-94.
20.Mackay IM. Clin Microbiol Infect 2004; 10:190-212.
21. Carvalho MG, et al. 2007, J Clin Microbiol 45:2460-2466
22. Morrison KE, et al., 2000 J Clin Microbiol 38:434-437
23. Hsu HE, et al., N Engl J Med. 2009 Jan 15;360(3):244-56.
24.Sandgren A, et al., 2004 J Infect Dis 189:785-796
25.Martell M, et al., J Clin Microbiol. 1999 Feb;37(2):327-32.;
26.Bièche I, et al., Int J Cancer. 1998 Nov 23;78(5):661-6.
27.Lallemand F, et al., J Clin Microbiol. 2000 Apr;38(4):1404-8).
28. Tarragó D, et al., Clin Microbiol Infect. 2008 Sep;14(9):828-34.
29. Scott JA, et al., 1996 Clin Infect Dis 22:973-981
30. McEllistrem MC, et al., J Infect Dis 2003; 188:1679-84
31.Byington CL, et al., Clinical Infectious Diseases 2005; 41:21-9
32.Finland M, et al., J Clin Microbiol 1977; 5:154-166
33.Butler JC, et al., J Infect Dis, 1995; 171:885-889.
34. Rubin LG, Rizvi A. et al., J. Med Microbiol 2004; 53: 595-602

## Claims

1. Method for detecting a specific serotype of *S. pneumoniae* in a sample, comprising the following steps:
a) extracting *S. pneumoniae* DNA from the sample;
b) incubating the extracted DNA under conditions such as to enable the amplification of at least one serotype specific target region comprised in the *Cps* gene cluster locus of *S. pneumoniae* by real-time PCR using at least SEQ ID NO. 4 and SEQ ID NO. 5 as forward and reverse primers and at least SEQ ID NO. 6 as probe sequence for serotype 3 if present in the sample, and
c) detecting and identifying the amplified product.

2. The method according to claim 1, wherein step b) enables the amplification of at least one further serotype specific target region comprised in the *Cps* gene cluster locus of *S. pneumoniae* selected from the group consisting of:
- SEQ ID No. 98 for serotype 1;
- SEQ ID No. 100 for serotype 4;
- SEQ ID No. 101 for serotype 5;
- SEQ ID No. 102 for serotype 6A/B;
- SEQ ID No. 103 for serotype 7A/F;
- SEQ ID No. 104 for serotype 8;
- SEQ ID No. 105 for serotype 9V/A;
- SEQ ID No. 106 for serotype 10A/B;
- SEQ ID No. 107 for serotype 12A/B/F;
- SEQ ID No. 108 for serotype 14;
- SEQ ID No. 109 for serotype 15;
- SEQ ID No. 110 for serotype 18B/C;
- SEQ ID No. 111 for serotype 19 B/F;
- SEQ ID No. 112 for serotype 19A;
- SEQ ID No. 113 for serotype 20;
- SEQ ID No. 114 for serotype 22F/A;
- SEQ ID No. 115 for serotype 23F;
- SEQ ID No. 116 for serotype 33 A/F;
- SEQ ID No. 117 for serotype 35B ;
- SEQ ID No. 118 for serotype 38 ;
- SEQ ID No. 119 for serotype 16F ;
- SEQ ID No. 120 for serotype 7B/7F ;
- SEQ ID No. 121 for serotype 35F (47F) ;
- SEQ ID No. 122 for serotype 17F ;
- SEQ ID No. 123 for serotype 23B ;
- SEQ ID No. 124 for serotype 23A ;
- SEQ ID No. 125 for serotype 29 ;
- SEQ ID No. 126 for serotype 2 ;
- SEQ ID No. 127 for serotype 11 ;
- SEQ ID No. 128 for serotype 21 ;
or
- SEQ ID No. 129 for serotype 24A/24F ;

3. The method according to claim 1 or 2 wherein the steps of amplification and detection occur by RT-PCR.

4. Kit for amplifying and detecting at least one serotype specific target region comprised in the *Cps* gene cluster locus of *S. pneumoniae* according to claim 1 or 2 comprising:
- at least one set of primer oligonucleotides and at least one probe oligonucleotide able to specifically amplify and detect at least one target region indicated in claim 1 or 2 wherein said primer oligonucleotides are at least SEQ ID NO. 4 and SEQ ID NO. 5 as forward and reverse primers and said probe is at least SEQ ID NO. 6 as probe sequence for serotype 3, and
- a positive control.

5. Kit according to claim 4 wherein the further set of primer oligonucleotides and the further probe oligonucleotide are selected from the group of:
- SEQ ID NO. 1 and 2 as forward and reverse primers and SEQ NO. 3 as probe sequence for serotype 1;
- SEQ ID NO. 7 and 8 as forward and reverse primers and SEQ NO. 9 as probe sequence for serotype 4;
- SEQ ID NO. 10 and 11 as forward and reverse primers and SEQ NO. 12 as probe sequence for serotype 5;
- SEQ ID NO. 13 and 14 as forward and reverse primers and SEQ NO. 15 as probe sequence for serotype 6A/B;
- SEQ ID NO. 16 and 17 as forward and reverse primers and SEQ NO. 18 as probe sequence for serotype 7F;
- SEQ ID NO. 19 and 20 as forward and reverse primers and SEQ NO. 21 as probe sequence for serotype 8;
- SEQ ID NO. 22 and 23 as forward and reverse primers and SEQ NO. 24 as probe sequence for serotype 9V/A;
- SEQ ID NO. 25 and 26 as forward and reverse primers and SEQ NO. 27 as probe sequence for serotype 10AF;
- SEQ ID NO. 28 and 29 as forward and reverse primers and SEQ NO. 30 as probe sequence for serotype 12ABF;
- SEQ ID NO. 31 and 32 as forward and reverse primers and SEQ NO. 33 as probe sequence for serotype 14;
- SEQ ID NO. 34 and 35 as forward and reverse primers and SEQ NO. 36 as probe sequence for serotype 15;
- SEQ ID NO. 37 and 38 as forward and reverse primers and SEQ NO. 39 as probe sequence for serotype 18B/C;
- SEQ ID NO. 40 and 41 as forward and reverse primers and SEQ NO. 42 as probe sequence for serotype 19A;
- SEQ ID NO. 43 and 44 as forward and reverse primers and SEQ NO. 45 as probe sequence for serotype 19 B/F;
- SEQ ID NO. 46 and 47 as forward and reverse primers and SEQ ID NO. 48 as probe sequence for serotype 20;
- SEQ ID NO. 49 and 50 as forward and reverse primers and SEQ ID NO. 51 as probe sequence for serotype 22F/A;
- SEQ ID NO. 52 and 53 as forward and reverse primers and SEQ ID NO. 54 as probe sequence for serotype 23F;
- SEQ ID NO. 55 and 56 as forward and reverse primers and SEQ ID NO. 57 as probe sequence for serotype 33 A/F;
- SEQ ID NO. 58 and 59 as forward and reverse primers and SEQ ID NO. 60 as probe sequence for serotype 35B;
- SEQ ID NO. 61 and 62 as forward and reverse primers and SEQ ID NO. 63 as probe sequence for serotype 38;
- SEQ ID NO. 65 and 66 as forward and reverse primers and SEQ ID NO. 67 as probe sequence for serotype 16F;
- SEQ ID NO. 68 and 69 as forward and reverse primers and SEQ ID NO. 70 as probe sequence for serotype 7B/7C;
- SEQ ID NO. 71 and 72 as forward and reverse primers and SEQ ID NO. 73 as probe sequence for serotype 35F (47F);
- SEQ ID NO. 74 and 75 as forward and reverse primers and SEQ ID NO. 76 as probe sequence for serotype 17F;
- SEQ ID NO. 77 and 78 as forward and reverse primers and SEQ ID NO. 79 as probe sequence for serotype 23B;
- SEQ ID NO. 80 and 81 as forward and reverse primers and SEQ ID NO. 82 as probe sequence for serotype 23A;
- SEQ ID NO. 83 and 84 as forward and reverse primer for serotype 29 and SEQ ID NO. 85 as probe sequence for serotype 29;
- SEQ ID NO. 86 and 87 as forward and reverse primers and SEQ ID NO. 88 as probe sequence for serotype 2;
- SEQ ID NO. 89 and 90 as forward and reverse primers and SEQ ID NO. 91 as probe sequence for serotype 11;
- SEQ ID NO. 92 and 93 as forward and reverse primers and SEQ ID NO. 94 as probe sequence for serotype 21;
- SEQ ID NO. 95 and 96 as forward and reverse primers and SEQ NO. 97 as probe sequence for serotype 24A/24F.

6. Kit according to claim 5 wherein the further set of primer oligonucleotides and the further probe oligonucleotide are selected from the group of:
- SEQ ID NO. 1 and 2 as forward and reverse primers and SEQ ID NO. 3 as probe sequence for serotype 1;
- SEQ ID NO. 7 and 8 as forward and reverse primers and SEQ NO. 9 as probe sequence for serotype 4;
- SEQ ID NO. 10 and 11 as forward and reverse primers and SEQ NO. 12 as probe sequence for serotype 5;
- SEQ ID NO. 13 and 14 as forward and reverse primers and SEQ NO. 15 as probe sequence for serotype 6A/B;
- SEQ ID NO. 16 and 17 as forward and reverse primers and SEQ NO. 18 as probe sequence for serotype 7F;
- SEQ ID NO. 19 and 20 as forward and reverse primers and SEQ NO. 21 as probe sequence for serotype 8;
- SEQ ID NO. 22 and 23 as forward and reverse primers and SEQ NO. 24 as probe sequence for serotype 9V/A;
- SEQ ID NO. 31 and 32 as forward and reverse primers and SEQ NO. 33 as probe sequence for serotype 14;
- SEQ ID NO. 34 and 35 as forward and reverse primers and SEQ NO. 36 as probe sequence for serotype 15;
- SEQ ID NO. 37 and 38 as forward and reverse primers and SEQ NO. 39 as probe sequence for serotype 18B/C;
- SEQ ID NO. 40 and 41 as forward and reverse primers and SEQ NO. 42 as probe sequence for serotype 19A;
- SEQ ID NO. 43 and 44 as forward and reverse primers and SEQ NO. 45 as probe sequence for serotype 19 B/F;
- SEQ ID NO. 49 and 50 as forward and reverse primers and SEQ NO. 51 as probe sequence for serotype 22F/A;
- SEQ ID NO. 52 and 53 as forward and reverse primers and SEQ NO. 54 as probe sequence for serotype 23F.

7. The kit according to any one of claim 5 or 6 wherein the set of primer oligonucleotides and the probe oligonucleotides for different serotypes are grouped in one or more reaction environments.

## Patentansprüche

1. Verfahren zum Nachweisen eines spezifischen Serotyps von *S. pneumoniae* in einer Probe, das die folgenden Schritte umfasst:
a) Extrahieren von *S. pneumoniae* DNA aus der Probe;
b) Inkubieren der extrahierten DNA unter Bedingungen, bei denen die Amplifikation von mindestens einer Serotyp-spezifischen Zielregion ermöglicht wird, die vom Cps-Genclusterlocus von *S. pneumoniae* umfasst wird, durch Echtzeit-PCR unter Verwendung von mindestens SEQ ID NO. 4 und SEQ ID NO. 5 als Vorwärts- und Rückwärtsprimer und mindestens SEQ ID NO. 6 als Sondensequenz für Serotyp 3, falls in der Probe vorhanden, und
c) Nachweisen und Identifizieren des amplifizierten Produkts.

2. Verfahren nach Anspruch 1, wobei Schritt b) die Amplifikation von mindestens einer weiteren Serotyp-spezifischen Zielregion ermöglicht, die von dem Cps-Genclusterlocus von *S. pneumoniae* umfasst wird, die ausgewählt wird aus der Gruppe bestehend aus:
- SEQ ID No. 98 für Serotyp 1;
- SEQ ID No. 100 für Serotyp 4;
- SEQ ID No. 101 für Serotyp 5;
- SEQ ID No. 102 für Serotyp 6A/B;
- SEQ ID No. 103 für Serotyp 7A/F;
- SEQ ID No. 104 für Serotyp 8;
- SEQ ID No. 105 für Serotyp 9V/A;
- SEQ ID No. 106 für Serotyp 10A/B;
- SEQ ID No. 107 für Serotyp 12A/B/F;
- SEQ ID No. 108 für Serotyp 14;
- SEQ ID No. 109 für Serotyp 15;
- SEQ ID No. 110 für Serotyp 18B/C;
- SEQ ID No. 111 für Serotyp 19B/F;
- SEQ ID No. 112 für Serotyp 19A;
- SEQ ID No. 113 für Serotyp 20;
- SEQ ID No. 114 für Serotyp 22F/A;
- SEQ ID No. 115 für Serotyp 23F;
- SEQ ID No. 116 für Serotyp 33A/F;
- SEQ ID No. 117 für Serotyp 35B;
- SEQ ID No. 118 für Serotyp 38;
- SEQ ID No. 119 für Serotyp 16F;
- SEQ ID No. 120 für Serotyp 7B/7F;
- SEQ ID No. 121 für Serotyp 35F(47F);
- SEQ ID No. 122 für Serotyp 17F;
- SEQ ID No. 123 für Serotyp 23B;
- SEQ ID No. 124 für Serotyp 23A;
- SEQ ID No. 125 für Serotyp 29;
- SEQ ID No. 126 für Serotyp 2;
- SEQ ID No. 127 für Serotyp 11;
- SEQ ID No. 128 für Serotyp 21;
oder
- SEQ ID No. 129 für Serotyp 24A/24F.

3. Verfahren nach Anspruch 1 oder 2, wobei die Schritte der Amplifikation und des Nachweises durch RT-PCR stattfinden.

4. Kit zum Amplifizieren und Nachweisen mindestens einer Serotyp-spezifischen Zielregion, die von dem Cps-Genclusterlocus von *S. pneumoniae* nach Anspruch 1 oder 2 umfasst wird, der Folgendes umfasst:
- mindestens einen Satz von Primer-Oligonukleotiden und mindestens ein Sonden-Oligonukleotid, die in der Lage sind, mindestens eine in Anspruch 1 oder 2 angegebene Zielregion spezifisch zu amplifizieren und nachzuweisen, wobei die Primer-Oligonukleotide mindestens SEQ ID NO. 4 und SEQ ID NO. 5 als Vorwärts- und Rückwärtsprimer sind und die Sonde mindestens SEQ ID NO. 6 als Sondensequenz für Serotyp 3 ist und
- eine Positivkontrolle.

5. Kit nach Anspruch 4, wobei der weitere Satz von Primer-Oligonukleotiden und das weitere Sonden-Oligonukleotid ausgewählt sind aus der Gruppe von:
- SEQ ID NO. 1 und 2 als Vorwärts- und Rückwärtsprimer und SEQ ID NO. 3 als Sondensequenz für Serotyp 1;
- SEQ ID NO. 7 und 8 als Vorwärts- und Rückwärtsprimer und SEQ ID NO. 9 als Sondensequenz für Serotyp 4;
- SEQ ID NO. 10 und 11 als Vorwärts- und Rückwärtsprimer und SEQ ID NO. 12 als Sondensequenz für Serotyp 5;
- SEQ ID NO. 13 und 14 als Vorwärts- und Rückwärtsprimer und SEQ ID NO. 15 als Sondensequenz für Serotyp 6A/B;
- SEQ ID NO. 16 und 17 als Vorwärts- und Rückwärtsprimer und SEQ ID NO. 18 als Sondensequenz für Serotyp 7F;
- SEQ ID NO. 19 und 20 als Vorwärts- und Rückwärtsprimer und SEQ ID NO. 21 als Sondensequenz für Serotyp 8;
- SEQ ID NO. 22 und 23 als Vorwärts- und Rückwärtsprimer und SEQ ID NO. 24 als Sondensequenz für Serotyp 9V/A;
- SEQ ID NO. 25 und 26 als Vorwärts- und Rückwärtsprimer und SEQ ID NO. 27 als Sondensequenz für Serotyp 10AF;
- SEQ ID NO. 28 und 29 als Vorwärts- und Rückwärtsprimer und SEQ ID NO. 30 als Sondensequenz für Serotyp 12ABF;
- SEQ ID NO. 31 und 32 als Vorwärts- und Rückwärtsprimer und SEQ ID NO. 33 als Sondensequenz für Serotyp 14;
- SEQ ID NO. 34 und 35 als Vorwärts- und Rückwärtsprimer und SEQ ID NO. 36 als Sondensequenz für Serotyp 15;
- SEQ ID NO. 37 und 38 als Vorwärts- und Rückwärtsprimer und SEQ ID NO. 39 als Sondensequenz für Serotyp 18B/C;
- SEQ ID NO. 40 und 41 als Vorwärts- und Rückwärtsprimer und SEQ ID NO. 42 als Sondensequenz für Serotyp 19A;
- SEQ ID NO. 43 und 44 als Vorwärts- und Rückwärtsprimer und SEQ ID NO. 45 als Sondensequenz für Serotyp 19B/F;
- SEQ ID NO. 46 und 47 als Vorwärts- und Rückwärtsprimer und SEQ ID NO. 48 als Sondensequenz für Serotyp 20;
- SEQ ID NO. 49 und 50 als Vorwärts- und Rückwärtsprimer und SEQ ID NO. 51 als Sondensequenz für Serotyp 22F/A;
- SEQ ID NO. 52 und 53 als Vorwärts- und Rückwärtsprimer und SEQ ID NO. 54 als Sondensequenz für Serotyp 23F;
- SEQ ID NO. 55 und 56 als Vorwärts- und Rückwärtsprimer und SEQ ID NO. 57 als Sondensequenz für Serotyp 33A/F;
- SEQ ID NO. 58 und 59 als Vorwärts- und Rückwärtsprimer und SEQ ID NO. 60 als Sondensequenz für Serotyp 35B;
- SEQ ID NO. 61 und 62 als Vorwärts- und Rückwärtsprimer und SEQ ID NO. 63 als Sondensequenz für Serotyp 38;
- SEQ ID NO. 65 und 66 als Vorwärts- und Rückwärtsprimer und SEQ ID NO. 67 als Sondensequenz für Serotyp 16F;
- SEQ ID NO. 68 und 69 als Vorwärts- und Rückwärtsprimer und SEQ ID NO. 70 als Sondensequenz für Serotyp 7B/7C;
- SEQ ID NO. 71 und 72 als Vorwärts- und Rückwärtsprimer und SEQ ID NO. 73 als Sondensequenz für Serotyp 35F(47F);
- SEQ ID NO. 74 und 75 als Vorwärts- und Rückwärtsprimer und SEQ ID NO. 76 als Sondensequenz für Serotyp 17F;
- SEQ ID NO. 77 und 78 als Vorwärts- und Rückwärtsprimer und SEQ ID NO. 79 als Sondensequenz für Serotyp 23B;
- SEQ ID NO. 80 und 81 als Vorwärts- und Rückwärtsprimer und SEQ ID NO. 82 als Sondensequenz für Serotyp 23A;
- SEQ ID NO. 83 und 84 als Vorwärts- und Rückwärtsprimer für Serotyp 29 und SEQ ID NO. 85 als Sondensequenz für Serotyp 29;
- SEQ ID NO. 86 und 87 als Vorwärts- und Rückwärtsprimer und SEQ ID NO. 88 als Sondensequenz für Serotyp 2;
- SEQ ID NO. 89 und 90 als Vorwärts- und Rückwärtsprimer und SEQ ID NO. 91 als Sondensequenz für Serotyp 11;
- SEQ ID NO. 92 und 93 als Vorwärts- und Rückwärtsprimer und SEQ ID NO. 94 als Sondensequenz für Serotyp 21;
- SEQ ID NO. 95 und 96 als Vorwärts- und Rückwärtsprimer und SEQ ID NO. 97 als Sondensequenz für Serotyp 24A/24F.

6. Kit nach Anspruch 5 wobei der weitere Satz von Primer-Oligonukleotiden und das weitere Sonden-Oligonukleotid ausgewählt sind aus der Gruppe von:
- SEQ ID NO. 1 und 2 als Vorwärts- und Rückwärtsprimer und SEQ ID NO. 3 als Sondensequenz für Serotyp 1;
- SEQ ID NO. 7 und 8 als Vorwärts- und Rückwärtsprimer und SEQ ID NO. 9 als Sondensequenz für Serotyp 4;
- SEQ ID NO. 10 und 11 als Vorwärts- und Rückwärtsprimer und SEQ ID NO. 12 als Sondensequenz für Serotyp 5;
- SEQ ID NO. 13 und 14 als Vorwärts- und Rückwärtsprimer und SEQ ID NO. 15 als Sondensequenz für Serotyp 6A/B;
- SEQ ID NO. 16 und 17 als Vorwärts- und Rückwärtsprimer und SEQ ID NO. 18 als Sondensequenz für Serotyp 7F;
- SEQ ID NO. 19 und 20 als Vorwärts- und Rückwärtsprimer und SEQ ID NO. 21 als Sondensequenz für Serotyp 8;
- SEQ ID NO. 22 und 23 als Vorwärts- und Rückwärtsprimer und SEQ ID NO. 24 als Sondensequenz für Serotyp 9V/A;
- SEQ ID NO. 31 und 32 als Vorwärts- und Rückwärtsprimer und SEQ ID NO. 33 als Sondensequenz für Serotyp 14;
- SEQ ID NO. 34 und 35 als Vorwärts- und Rückwärtsprimer und SEQ ID NO. 36 als Sondensequenz für Serotyp 15;
- SEQ ID NO. 37 und 38 als Vorwärts- und Rückwärtsprimer und SEQ ID NO. 39 als Sondensequenz für Serotyp 18B/C;
- SEQ ID NO. 40 und 41 als Vorwärts- und Rückwärtsprimer und SEQ ID NO. 42 als Sondensequenz für Serotyp 19A;
- SEQ ID NO. 43 und 44 als Vorwärts- und Rückwärtsprimer und SEQ ID NO. 45 als Sondensequenz für Serotyp 19B/F;
- SEQ ID NO. 49 und 50 als Vorwärts- und Rückwärtsprimer und SEQ ID NO. 51 als Sondensequenz für Serotyp 22F/A;
- SEQ ID NO. 52 und 53 als Vorwärts- und Rückwärtsprimer und SEQ ID NO. 54 als Sondensequenz für Serotyp 23F.

7. Kit nach einem der Ansprüche 5 oder 6, wobei der Satz von Primer-Oligonukleotiden und die Sonden-Oligonukleotide für verschiedene Serotypen in einer oder mehreren Reaktionsumgebungen gruppiert sind.

## Revendications

1. Procédé de détection d'un sérotype spécifique de *S. Pneumoniae* dans un échantillon, comprenant les étapes suivantes :
a) extraction de l'ADN de *S. Pneumoniae* à partir de l'échantillon ;
b) incubation de l'ADN extrait dans des conditions permettant l'amplification d'au moins une région cible spécifique du sérotype comprise dans le locus du groupe de gènes *Cps* de *S. Pneumoniae* par PCR en temps réel en utilisant au moins SEQ ID n° 4 et SEQ ID n° 5 en tant qu'amorces sens et antisens et au moins SEQ ID n° 6 en tant que séquence sonde pour le sérotype 3 s'il est présent dans l'échantillon, et
c) détection et identification du produit amplifié.

2. Procédé selon la revendication 1, dans lequel l'étape b) permet l'amplification d'au moins une ultérieure région cible spécifique du sérotype comprise dans le locus du groupe de gènes *Cps* de *S. Pneumoniae* choisie dans le groupe constitué par :
- SEQ ID n° 98 pour le sérotype 1 ;
- SEQ ID n° 100 pour le sérotype 4 ;
- SEQ ID n° 101 pour le sérotype 5 ;
- SEQ ID n° 102 pour le sérotype 6A/B ;
- SEQ ID n° 103 pour le sérotype 7A/F ;
- SEQ ID n° 104 pour le sérotype 8 ;
- SEQ ID n° 105 pour le sérotype 9V/A ;
- SEQ ID n° 106 pour le sérotype 10A/B ;
- SEQ ID n° 107 pour le sérotype 12A/B/F ;
- SEQ ID n° 108 pour le sérotype 14 ;
- SEQ ID n° 109 pour le sérotype 15 ;
- SEQ ID n° 110 pour le sérotype 18B/C ;
- SEQ ID n° 111 pour le sérotype 19B/F ;
- SEQ ID n° 112 pour le sérotype 19A ;
- SEQ ID n° 113 pour le sérotype 20 ;
- SEQ ID n° 114 pour le sérotype 22F/A ;
- SEQ ID n° 115 pour le sérotype 23F ;
- SEQ ID n° 116 pour le sérotype 33A/F ;
- SEQ ID n° 117 pour le sérotype 35B ;
- SEQ ID n° 118 pour le sérotype 38 ;
- SEQ ID n° 119 pour le sérotype 16F ;
- SEQ ID n° 120 pour le sérotype 7B/7F ;
- SEQ ID n° 121 pour le sérotype 35F (47F) ;
- SEQ ID n° 122 pour le sérotype 17F ;
- SEQ ID n° 123 pour le sérotype 23B ;
- SEQ ID n° 124 pour le sérotype 23A ;
- SEQ ID n° 125 pour le sérotype 29 ;
- SEQ ID n° 126 pour le sérotype 2 ;
- SEQ ID n° 127 pour le sérotype 11 ;
- SEQ ID n° 128 pour le sérotype 21 ;
ou
- SEQ ID n° 129 pour le sérotype 24A/24F.

3. Procédé selon la revendication 1 ou 2, dans lequel les étapes d'amplification et de détection sont réalisées par RT-PCR.

4. Kit d'amplification et de détection d'au moins une région cible spécifique du sérotype comprise dans le locus du groupe de gène *Cps* de *S. Pneumoniae* selon la revendication 1 ou 2 comprenant :
- au moins un groupe d'oligonucléotides amorces et au moins un oligonucléotide sonde capables d'amplifier et de détecter spécifiquement au moins une région cible indiquée dans la revendication 1 ou 2, dans lequel lesdits oligonucléotides amorces sont au moins SEQ ID n° 4 et SEQ ID n° 5 en tant qu'amorces sens et antisens et ladite sonde est au moins SEQ ID n° 6 en tant que séquence sonde pour le sérotype 3, et
- un contrôle positif.

5. Kit selon la revendication 4, dans lequel l'ulterieur groupe d'oligonucléotides amorces et l'ulterieur oligonucléotide sonde sont choisis dans le groupe constitué par :
- SEQ ID n° 1 et 2 en tant qu'amorces sens et antisens et SEQ ID n° 3 en tant que séquence sonde pour le sérotype 1 ;
- SEQ ID n° 7 et 8 en tant qu'amorces sens et antisens et SEQ ID n° 9 en tant que séquence sonde pour le sérotype 4 ;
- SEQ ID n° 10 et 11 en tant qu'amorces sens et antisens et SEQ ID n° 12 en tant que séquence sonde pour le sérotype 5 ;
- SEQ ID n° 13 et 14 en tant qu'amorces sens et antisens et SEQ ID n° 15 en tant que séquence sonde pour le sérotype 6A/B ;
- SEQ ID n° 16 et 17 en tant qu'amorces sens et antisens et SEQ ID n° 18 en tant que séquence sonde pour le sérotype 7F ;
- SEQ ID n° 19 et 20 en tant qu'amorces sens et antisens et SEQ ID n° 21 en tant que séquence sonde pour le sérotype 8 ;
- SEQ ID n° 22 et 23 en tant qu'amorces sens et antisens et SEQ ID n° 24 en tant que séquence sonde pour le sérotype 9V/A ;
- SEQ ID n° 25 et 26 en tant qu'amorces sens et antisens et SEQ ID n° 27 en tant que séquence sonde pour le sérotype 10AF ;
- SEQ ID n° 28 et 29 en tant qu'amorces sens et antisens et SEQ ID n° 30 en tant que séquence sonde pour le sérotype 12ABF ;
- SEQ ID n° 31 et 32 en tant qu'amorces sens et antisens et SEQ ID n° 33 en tant que séquence sonde pour le sérotype 14 ;
- SEQ ID n° 34 et 35 en tant qu'amorces sens et antisens et SEQ ID n° 36 en tant que séquence sonde pour le sérotype 15 ;
- SEQ ID n° 37 et 38 en tant qu'amorces sens et antisens et SEQ ID n° 39 en tant que séquence sonde pour le sérotype 18B/C ;
- SEQ ID n° 40 et 41 en tant qu'amorces sens et antisens et SEQ ID n° 42 en tant que séquence sonde pour le sérotype 19A ;
- SEQ ID n° 43 et 44 en tant qu'amorces sens et antisens et SEQ ID n° 45 en tant que séquence sonde pour le sérotype 19B/F ;
- SEQ ID n° 46 et 47 en tant qu'amorces sens et antisens et SEQ ID n° 48 en tant que séquence sonde pour le sérotype 20 ;
- SEQ ID n° 49 et 50 en tant qu'amorces sens et antisens et SEQ ID n° 51 en tant que séquence sonde pour le sérotype 22F/A ;
- SEQ ID n° 52 et 53 en tant qu'amorces sens et antisens et SEQ ID n° 54 en tant que séquence sonde pour le sérotype 23F ;
- SEQ ID n° 55 et 56 en tant qu'amorces sens et antisens et SEQ ID n° 57 en tant que séquence sonde pour le sérotype 33A/F ;
- SEQ ID n° 58 et 59 en tant qu'amorces sens et antisens et SEQ ID n° 60 en tant que séquence sonde pour le sérotype 35B ;
- SEQ ID n° 61 et 62 en tant qu'amorces sens et antisens et SEQ ID n° 63 en tant que séquence sonde pour le sérotype 38 ;
- SEQ ID n° 65 et 66 en tant qu'amorces sens et antisens et SEQ ID n° 67 en tant que séquence sonde pour le sérotype 16F ;
- SEQ ID n° 68 et 69 en tant qu'amorces sens et antisens et SEQ ID n° 70 en tant que séquence sonde pour le sérotype 7B/7C ;
- SEQ ID n° 71 et 72 en tant qu'amorces sens et antisens et SEQ ID n° 73 en tant que séquence sonde pour le sérotype 35F (47F) ;
- SEQ ID n° 74 et 75 en tant qu'amorces sens et antisens et SEQ ID n° 76 en tant que séquence sonde pour le sérotype 17F ;
- SEQ ID n° 77 et 78 en tant qu'amorces sens et antisens et SEQ ID n° 79 en tant que séquence sonde pour le sérotype 23B ;
- SEQ ID n° 80 et 81 en tant qu'amorces sens et antisens et SEQ ID n° 82 en tant que séquence sonde pour le sérotype 23A ;
- SEQ ID n° 83 et 84 en tant qu'amorces sens et antisens pour le sérotype 29 et SEQ ID n° 85 en tant que séquence sonde pour le sérotype 29 ;
- SEQ ID n° 86 et 87 en tant qu'amorces sens et antisens et SEQ ID n° 88 en tant que séquence sonde pour le sérotype 2 ;
- SEQ ID n° 89 et 90 en tant qu'amorces sens et antisens et SEQ ID n° 91 en tant que séquence sonde pour le sérotype 11 ;
- SEQ ID n° 92 et 93 en tant qu'amorces sens et antisens et SEQ ID n° 94 en tant que séquence sonde pour le sérotype 21 ;
- SEQ ID n° 95 et 96 en tant qu'amorces sens et antisens et SEQ ID n° 97 en tant que séquence sonde pour le sérotype 24A/24F.

6. Kit selon la revendication 5, dans lequel l'ulterieur groupe d'oligonucléotides amorces et l'ulterieur oligonucléotide sonde sont choisis dans le groupe constitué par :
- SEQ ID n° 1 et 2 en tant qu'amorces sens et antisens et SEQ ID n° 3 en tant que séquence sonde pour le sérotype 1 ;
- SEQ ID n° 7 et 8 en tant qu'amorces sens et antisens et SEQ ID n° 9 en tant que séquence sonde pour le sérotype 4 ;
- SEQ ID n° 10 et 11 en tant qu'amorces sens et antisens et SEQ ID n° 12 en tant que séquence sonde pour le sérotype 5 ;
- SEQ ID n° 13 et 14 en tant qu'amorces sens et antisens et SEQ ID n° 15 en tant que séquence sonde pour le sérotype 6A/B ;
- SEQ ID n° 16 et 17 en tant qu'amorces sens et antisens et SEQ ID n° 18 en tant que séquence sonde pour le sérotype 7F ;
- SEQ ID n° 19 et 20 en tant qu'amorces sens et antisens et SEQ ID n° 21 en tant que séquence sonde pour le sérotype 8 ;
- SEQ ID n° 22 et 23 en tant qu'amorces sens et antisens et SEQ ID n° 24 en tant que séquence sonde pour le sérotype 9V/A ;
- SEQ ID n° 31 et 32 en tant qu'amorces sens et antisens et SEQ ID n° 33 en tant que séquence sonde pour le sérotype 14 ;
- SEQ ID n° 34 et 35 en tant qu'amorces sens et antisens et SEQ ID n° 36 en tant que séquence sonde pour le sérotype 15 ;
- SEQ ID n° 37 et 38 en tant qu'amorces sens et antisens et SEQ ID n° 39 en tant que séquence sonde pour le sérotype 18B/C ;
- SEQ ID n° 40 et 41 en tant qu'amorces sens et antisens et SEQ ID n° 42 en tant que séquence sonde pour le sérotype 19A ;
- SEQ ID n° 43 et 44 en tant qu'amorces sens et antisens et SEQ ID n° 45 en tant que séquence sonde pour le sérotype 19B/F ;
- SEQ ID n° 49 et 50 en tant qu'amorces sens et antisens et SEQ ID n° 51 en tant que séquence sonde pour le sérotype 22F/A ;
- SEQ ID n° 52 et 53 en tant qu'amorces sens et antisens et SEQ ID n° 54 en tant que séquence sonde pour le sérotype 23F.

7. Kit selon l'une quelconque des revendications 5 ou 6, dans lequel le groupe d'oligonucléotides amorces et les oligonucléotides sondes pour différents sérotypes sont regroupés dans un ou plusieurs milieux réactionnels.
